(19) **Europäisches Patentamt / European Patent Office / Office européen des brevets**

(11) **EP 4 603 472 A1**

(12) **EUROPEAN PATENT APPLICATION**
published in accordance with Art. 153(4) EPC

(43) Date of publication:
20.08.2025 Bulletin 2025/34

(21) Application number: 23877208.1

(22) Date of filing: 04.10.2023

(51) International Patent Classification (IPC):
$C07C\ 45/35^{(2006.01)}$    $B01J\ 23/887^{(2006.01)}$
$B01J\ 35/51^{(2024.01)}$    $C07B\ 61/00^{(2006.01)}$
$C07C\ 47/22^{(2006.01)}$

(52) Cooperative Patent Classification (CPC):
B01J 23/887; B01J 35/51; C07B 61/00;
C07C 45/35; C07C 47/22

(86) International application number:
PCT/JP2023/036202

(87) International publication number:
WO 2024/080203 (18.04.2024 Gazette 2024/16)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 12.10.2022 JP 2022163792

(71) Applicant: **Nippon Kayaku Kabushiki Kaisha Tokyo 100-0005 (JP)**

(72) Inventors:
- **OKUMURA, Shigeki**
  **Sanyoonoda-shi, Yamaguchi 757-8686 (JP)**
- **KAGAWA, Tsukasa**
  **Sanyoonoda-shi, Yamaguchi 757-8686 (JP)**
- **KAWAMURA, Tomoyuki**
  **Tokyo 100-0005 (JP)**

(74) Representative: **Gille Hrabal Partnerschaftsgesellschaft mbB Patentanwälte Brucknerstraße 20 40593 Düsseldorf (DE)**

(54) **METHOD FOR PRODUCING UNSATURATED ALDEHYDE, AND APPARATUS FOR PRODUCING UNSATURATED ALDEHYDE**

(57)    The present invention relates to a method for producing an unsaturated aldehyde corresponding to an alkene including partially oxidizing the alkene using a fixed-bed multitubular reactor. The fixed-bed multitubular reactor includes multiple reaction tubes and a reaction bath for adjusting temperature of the multiple reaction tubes. The reaction tube is provided with two or more catalyst layers in a gas flow direction. When a reaction bath temperature at which a yield of the unsaturated aldehyde is the highest is defined as A (°C), and reaction bath temperatures at which the yield is 1.0% lower than the highest value are defined as A1 (°C) and A2 (°C), following formulae (1) and (2) hold:

$$A1 < A < A2 \quad (1);$$

and

$$(A2 - A1) \geq 10 \quad (2).$$

EP 4 603 472 A1

**Description**

TECHNICAL FIELD

The present invention relates to a method and a device for producing an unsaturated aldehyde corresponding to an alkene by subjecting the alkene to gas-phase catalytic oxidation with molecular oxygen or a gas containing molecular oxygen.

BACKGROUND ART

Methods for producing an unsaturated aldehyde corresponding to an alkene by using the alkene or an alcohol that may produce alkenes through the intramolecular dehydration reaction as a raw material are widely used industrially. In particular, many proposals have been made in the past regarding catalysts for synthesizing acrolein by gas-phase catalytic oxidation of propylene with molecular oxygen.

In this gas-phase oxidation reaction, the yield is the most important consideration from the viewpoint of productivity. Therefore, Patent Literature 1 describes a technique related to the atomic ratio between iron, and cobalt and nickel as an improvement of the compositional components of the catalyst. Patent Literature 2 describes a technique related to the atomic ratio of iron to cobalt and/or nickel. Patent Literature 3 describes a technique related to the atomic ratios of nickel to bismuth, nickel to an alkali metal component, and bismuth to an alkali metal component, as well as optimization of the atomic ratios of each element to molybdenum. In addition, Patent Literature 4 describes an improvement to the compositional ratio of bismuth to molybdenum.

In addition, this reaction system is accompanied by intense heat generation so that the occurrence of local high-temperature portions (hot spots) in a catalyst layer is a major problem. The "hot spot" generally refers to the maximum value of the temperature in the catalyst layer, it usually occurs in the catalyst layer located on the gas inlet side where the raw material concentration is high, but it can also occur in the highly active catalyst layer located on the gas outlet side due to deactivation of the catalyst on the inlet side, sudden disturbance factors, or variations in various conditions. As used herein, the "disturbance factor" refers to, for example, changes in the flow rate of the heat transfer medium supplied to the reaction bath jacket, and fluctuations in the flow rate of the raw material gas due to air temperature.

The occurrence of the hot spot leads to a shortened catalyst life, a decrease in yield due to excessive oxidation reactions, and in some cases, runaway reactions. Therefore, several technologies have been proposed to control the activity of the catalysts filled into the portions where the hot spot occurs in order to suppress the hot spot temperature.

For example, Patent Literature 5 discloses a technique for lowering the hot spot temperature by using a catalyst whose activity is adjusted by changing the loading amount and a catalyst whose activity is adjusted by changing the calcining temperature of the catalyst. Patent Literature 6 discloses a technique for using a catalyst whose activity is adjusted by changing the ratio of the apparent densities of the catalyst. Patent Literature 7 discloses a technique for using a catalyst whose activity is adjusted by changing the content of inactive components in the catalyst molded body as well as changing the volume occupied by the catalyst molded body, the type and/or amount of alkali metals, and the calcining temperature of the catalyst. Patent Literature 8 discloses a technique for providing reactive areas with different volumes occupied by a catalyst molded body and mixing inactive substances into at least one of the reactive areas. Patent Literature 9 discloses a technique for using a catalyst whose activity is adjusted by changing the calcining temperature of the catalyst. Patent Literature 10 discloses a technique for using a catalyst whose activity is adjusted by changing the volume occupied by the catalyst, the calcining temperature, and/or the type and amount of the alkali metals.

CITATION LIST

PATENT LITERATURE

Patent Literature 1 Japanese Unexamined Patent Application Publication No. 2003-164763
Patent Literature 2 Japanese Unexamined Patent Application Publication No. 2003-146920
Patent Literature 3 International Publication No. WO2014/181839
Patent Literature 4 International Publication No. WO2016/136882
Patent Literature 5 Japanese Unexamined Patent Application Publication No. H10-168003
Patent Literature 6 Japanese Unexamined Patent Application Publication No. 2004-002209
Patent Literature 7 Japanese Unexamined Patent Application Publication No. 2001-328951
Patent Literature 8 Japanese Unexamined Patent Application Publication No. 2005-320315
Patent Literature 9 Japanese Unexamined Patent Application Publication No. H08-3093

Patent Literature 10 Japanese Unexamined Patent Application Publication No. 2001-226302
Patent Literature 11 Japanese Patent No. 6912153

SUMMARY OF INVENTION

TECHNICAL PROBLEM

However, even if the yield is improved by the aforementioned means, the yield of the target product is not sufficient. Since it affects the amount of the alkene required for production and has a great impact on production costs, improvements are required. In addition, by continuing operation at a low yield, there arises a problem that a large amount of by-products produced places a heavy burden on the refining process, resulting in increased time and operating costs for the refining process. Furthermore, depending on the type of by-products, they may be deposited on the surface of the catalyst or in the gas flow path near the catalyst and cover necessary reactive active points on the surface of the catalyst, resulting in reduced activity of the catalyst, which makes it necessary to forcibly increase the activity. Therefore, the temperature of the reaction bath has to be increased. This causes the catalyst to be subjected to thermal stress, shortening its lifespan and reducing the selectivity, which leads to a further decrease in yield.

Furthermore, measures to ensure stable operation including the suppression of the hot spots, are still insufficient. For example, in industrial plants, variations in the heat removal capacity due to the structure of the reactors, distribution of the heat transfer medium temperature in the horizontal and vertical directions, and distribution of the gas flow rate for each reaction tube can occur. Therefore, it is almost impossible to use the catalyst under the same conditions in all reaction tubes.

When analyzing catalysts having been used in industrial plants, there can be found reaction tubes in which the catalyst in the raw material gas inlet portion is deteriorated intensively, reaction tubes in which the catalyst is moderately deteriorated throughout, or even more surprising reactor tubes in which the catalyst in the raw material gas outlet portion is more deteriorated than the catalyst in the inlet portion. The last one suggests the hot spot temperature of the catalyst layer on the raw material gas outlet side may have been abnormally high, which may lead to runaway reactions in some cases. The cause thereof is presumed to be the difference in the conversion rate of the feedstock hydrocarbons, and thus the difference in the shapes of temperature distribution, which are due to variations in the diameter of the reaction tubes, variations in the heat removal capacity due to the structure of the reactors, distribution of the heat transfer medium temperature in the horizontal and vertical directions, and distribution of the gas flow rate for each reaction tube in the aforementioned industrial plants. The challenge was to develop a technique for maintaining the reaction more safely and stably for a long period of time even when various factors of variation occur at the same time. If a plant shuts down due to an abnormal reaction, losses will occur due to unproduction during the relevant period, and abnormal temperatures or atmospheres may shorten the catalyst lifespan. Moreover, if a runaway reaction occurs, it could lead to significant losses such as damage to the industrial plant equipment itself, and an accident. Thus, there is a need for an improvement for ensuring stable operation for maintain a stable yield over a wide range of the reaction bath temperature. This is especially true for multitubular reactors, which have multiple reaction tubes.

A multi-layer packing technique has been used as a conventional production method, in which a catalyst with low activity is used on the gas inlet side of the reaction tube, a catalyst with high activity is used on the gas outlet side of the reaction tube, and a packing length of a catalyst layer near the gas outlet side is made longer than the gas inlet side.

In particular, a method for improving a yield of acrolein in a method for producing acrolein from propylene is described in, for example, Patent Literature 11. However, another problem may occur here. As described above, it is common to use a catalyst on the outlet side of the reaction gas by multi-layer packing of catalysts, and here, from the viewpoint of stable operation of the plant, it is an important issue whether the hot spot temperature (PTf) of the most active catalyst on the outlet side changes steeply with respect to the reaction bath temperature (BT). This is because, when the PTf changes steeply with respect to the BT ($\Delta$PTf/$\Delta$BT is large), a slight change of the reaction bath temperature or a difference in the reaction bath temperature between multiple reaction tubes causes a steep change in the hot spot on the most active catalyst on the outlet side, which makes it more likely to cause a thermal runaway and the resulting damage or explosion of the reaction tubes. In addition, the high PTf may lead to: a decrease in a yield of acrolein due to a cool flame reaction (cool flame) of acrolein which is caused by the fact that a large amount of heat is generated at the outlet side of the reactor and makes the gas temperature high at the outlet of the reactor; deposit of carbonaceous precipitates on the outlet side of the reaction tubes due to an autoxidation reaction of acrolein (coking or fouling); and blockage inside the reaction tubes due to the latter. As a background to the aforementioned problems, the reason why $\Delta$PTf/$\Delta$BT becomes large in the method for producing acrolein from propylene is described below. When producing acrolein from propylene, a reactor and a process are designed so that sequential oxidation does not occur because acrylic acid, which is a product of sequential oxidation, is considered as a by-product. For example: (1) a high load condition is utilized because gas convection and sequential oxidation would occur with a low load (= low space velocity of the raw material, propylene), (2) a packing amount of inert materials placed at the raw material gas inlet portion is reduced; (3) an oxygen/propylene ratio at an inlet of a catalyst layer is set low in order to suppress the decrease in the yield of acrolein due to the successive oxidation reaction; (4) an outlet pressure is set high to compensate for a decrease in the overall catalytic activity due to the above (1) to (3); and the like. Because of these factors, particularly in the method for producing acrolein from propylene, PTf is likely to be the highest temperature in all the catalyst layers made by multi-layer packing, and PTf is likely to change steeply with respect to BT.

The specific calculation method of $\Delta PTf/\Delta BT$ is as follows. When all of three or more measurement points, at which the peak temperature (PTf) closest to the outlet and BT within a range between A1 and A2 described below are actually measured, are plotted as a scatter plot, $\Delta PTf/\Delta BT$ is defined as a slope of an approximate straight line obtained by the least squares method, which has an intercept on the vertical axis representing the peak temperature (PTf) closest to the outlet and is assumed to be a linear function of BT. When there are only two measurement points, a slope of a straight line passing through these two points is set as $\Delta PTf/\Delta BT$. The measurement points are set so that the difference (BT2 - BT1) between the reaction bath temperature BT1 at a measurement point with the lowest reaction bath temperature (BT) and the reaction bath temperature BT2 at a measurement point with the highest reaction bath temperature is 30% or more of A2 - A1.

As described above, particularly in the method for producing acrolein from propylene, a catalyst, a method for packing the catalyst, and a method for managing the operation that are to be adopted for stable operation of a plant under the above-mentioned restrictions on the reactor and process, have not been known. Furthermore, a catalyst and a method for packing the catalyst that are preferable for stable operation focusing on the peak temperature closest to the outlet have not been known.

SOLUTION TO PROBLEM

As a result of thorough consideration of the aforementioned current situation and issues, the present inventors found that stable plant operation with a high yield can be achieved by packing a catalyst so as to widen a reaction bath temperature zone in which a yield of acrolein is stable (hereinafter, referred to as an operation window). In addition, they also found that when packing a catalyst so as to establish a certain relationship between the sum of spot heat generation temperatures in a catalyst layer closest to an outlet and the sum of spot heat generation temperatures in all catalyst layers, PTf can change gently with respect to BT ($\Delta PTf/\Delta BT$ can be reduced), and that a yield of an unsaturated aldehyde is increased and stable plant operation with a higher yield can be achieved. The present invention can be applied not only to a reaction from propylene to acrolein but also to a reaction from tert-butyl alcohol and/or isobutylene to methacrolein, for example.

That is, the present invention relates to the following 1) to 11).

1) A method for producing an unsaturated aldehyde corresponding to an alkene including partially oxidizing the alkene using a fixed-bed multitubular reactor, in which

the fixed-bed multitubular reactor comprises multiple reaction tubes and a reaction bath for adjusting temperature of the multiple reaction tubes,
the reaction tube is provided with two or more catalyst layers in a gas flow direction, and
when a reaction bath temperature at which a yield of the unsaturated aldehyde is the highest is defined as A (°C), and reaction bath temperatures at which the yield is 1.0% lower than the highest value are defined as A1 (°C) and A2 (°C), following formulae (1) and (2) hold:

$$A1 < A < A2 \qquad (1)$$

$$(A2 - A1) \geq 10 \qquad (2).$$

2) The method for producing the unsaturated aldehyde according to 1), in which

regarding Sf and St when the unsaturated aldehyde is produced at the reaction bath temperature A (°C), a following formula (3) holds:

$$(Sf/St) \times 100 \leq 42.0 \qquad (3),$$

and Sf and St are determined by following steps (a) to (c):

(a) p (p is an integer of 2 or more) thermocouples are arranged at regular intervals throughout the gas flow direction of the two or more catalyst layers provided in the reaction tube, and spot temperatures $T1_j$ (°C) (j is 1 to p) are obtained by using the thermocouples at p measurement points in the reaction tube when producing the unsaturated aldehyde at the reaction bath temperature A (°C);

(b) for each of the measurement points, a spot heat generation temperature $T2_j$, which is a value ($T1_j$ - A) obtained by subtracting the reaction bath temperature A (°C) from the spot temperature $T1_j$ (°C), is calculated, provided that if $T1_j$ - A is a negative value, the spot heat generation temperature $T2_j$ of the relevant measurement point is set to 0; and

(c) St is defined as a value of the sum of the spot heat generation temperatures $T2_j$ at all the measurement points in the reaction tube, and Sf is defined as a value of the sum of the spot heat generation temperatures $T2_k$ at the measurement points provided in the catalyst layer closest to an outlet, where k is 1 to q, and q is number of the measurement points provided in the catalyst layer closest to the outlet, q < p.

3) The method for producing the unsaturated aldehyde according to 1) or 2), in which

a catalytically active component contained in the catalyst layer closest to an inlet of a reaction raw material gas has composition represented by a following formula (I-1):

$$Mo_{a1}Bi_{b1}Ni_{c1}CO_{d1}Fe_{e1}X_{f1}Cs_{g1}Z_{h1}O_{i1} \qquad (I-1),$$

where, Mo, Bi, Ni, Co, Fe, Cs, and O represent molybdenum, bismuth, nickel, cobalt, iron, cesium, and oxygen, respectively;

X represents at least one element selected from tungsten, antimony, tin, zinc, chromium, manganese, magnesium, silicon, aluminum, cerium, and titanium;

Z represents at least one element belonging to groups 1 to 16 on the periodic table and selected from elements other than Mo, Bi, Ni, Co, Fe, Cs, O, and X described above; and

a1, b1, c1, d1, e1, f1, g1, h1, and i1 represent number of atoms of molybdenum, bismuth, nickel, cobalt, iron, X, Cs, Z, and oxygen, respectively, in which, when a1 = 12, $0 < b1 \leq 7.0$, $0 \leq c1 \leq 10$, $0 < d1 \leq 10$, $0 < e1 \leq 5.0$, $0 \leq f1 \leq 2.0$, $0 < g1 \leq 3.0$, and $0 \leq h1 \leq 5.0$ are satisfied, and i1 is a value determined by an oxidation state of each element.

4) The method for producing the unsaturated aldehyde according to 1) or 2), in which

a catalytically active component contained in the catalyst layer closest to the outlet of the reaction raw material gas has composition represented by a following formula (I-2):

$$Mo_{a2}Bi_{b2}Ni_{c2}Co_{d2}Fe_{e2}X_{f2}K_{g2}Z_{h2}O_{i2} \qquad (I-2)$$

where, Mo, Bi, Ni, Co, Fe, and K represent molybdenum, bismuth, nickel, cobalt, iron, and potassium, respectively;

X represents at least one element selected from tungsten, antimony, tin, zinc, chromium, manganese, magnesium, silicon, aluminum, cerium, and titanium;

Z represents at least one element belonging to groups 1 to 16 on the periodic table and selected from elements other than Mo, Bi, Ni, Co, Fe, K, O, and X described above; and

a2, b2, c2, d2, e2, f2, g2, h2, and i2 represent number of atoms of molybdenum, bismuth, nickel, cobalt, iron, X, K, Z, and oxygen, respectively, in which, when a2 = 12, $0 < b2 \leq 7.0$, $0 \leq c2 \leq 10$, $0 < d2 \leq 10$, $0 < e2 \leq 5.0$, $0 \leq f2 \leq 2.0$, $0 \leq g2 \leq 3.0$, and $0 \leq h2 \leq 5.0$ are satisfied, and i2 is a value determined by an oxidation state of each element.

5) The method for producing the unsaturated aldehyde according to 1) or 2), in which a reaction bath temperature is 310°C or higher and 340°C or lower.

6) The method for producing the unsaturated aldehyde according to 1) or 2), in which a volume ratio of oxygen to the alkene (oxygen/alkene) in a reaction raw material gas is 1.0 or more and 1.8 or less.

7) The method for producing the unsaturated aldehyde according to 1) or 2), in which the reaction tube does not have an inert layer closer to the inlet than the catalyst layers.

8) The method for producing the unsaturated aldehyde according to 1) or 2), in which

the reaction tube is provided with three catalyst layers in the gas flow direction, and a ratio of the sum of packing lengths of a first layer and a second layer counted from an inlet side of the reaction tube to a packing length of a third layer ((packing length of first layer + packing length of second layer)/packing length of third layer) is 1.5 or more and 3.5 or less.

9) The method for producing the unsaturated aldehyde according to 1) or 2), in which

the reaction tube is provided with two or more catalyst layers in the gas flow direction, and a type, dilution ratio, and packing length of the catalyst in each of the catalyst layers are set so that akt/akn defined by following formulae (II), (III), and (IV) is 1.41 or more and 10.00 or less:

$$\text{Reaction rate k of catalyst} = -\text{Ln}\,(1 - x/100) \quad (II)$$

$$\text{Actual reaction rate ak of catalyst layer} = (\text{dilution ratio of relevant catalyst layer}) \times (\text{packing length of relevant catalyst layer}) \times (\text{reaction rate k of catalyst in relevant catalyst layer}) \quad (III)$$

$$\text{akt/akn} = (\text{value of the sum of actual reaction rates ak of all catalyst layers})/(\text{actual reaction rate ak of catalyst layer closest to outlet of reaction tube}) \quad (IV)$$

in which x represents a raw material gas conversion ratio (%) when the catalyst is packed into a differential reactor and a partial oxidation reaction of the alkene is conducted at the reaction bath temperature of 360°C.

10) A production device of an unsaturated aldehyde by partially oxidizing an alkene to produce a corresponding unsaturated aldehyde, including:

a fixed-bed multitubular reactor including multiple reaction tubes; and
a reaction bath for adjusting temperature of the multiple reaction tubes, wherein
the reaction tube is provided with two or more catalyst layers in a gas flow direction, and
when a reaction bath temperature at which a yield of the unsaturated aldehyde is the highest is defined as A (°C), and reaction bath temperatures at which the yield is 1.0% lower than the highest value are defined as A1 (°C) and A2 (°C), following formulae (1) and (2) hold:

$$A1 < A < A2 \quad (1)$$

$$(A2 - A1) \geq 10 \quad (2)$$

11) A method for producing an unsaturated aldehyde corresponding to an alkene comprising partially oxidizing the alkene using a fixed-bed multitubular reactor, in which

the fixed-bed multitubular reactor includes multiple reaction tubes and a reaction bath for adjusting temperature of the multiple reaction tubes,
the reaction tube is provided with two or more catalyst layers in a gas flow direction,
regarding Sf and St when the unsaturated aldehyde is produced at a reaction bath temperature A (°C) at which a yield of the unsaturated aldehyde is the highest, a following formula (3) holds:

$$(\text{Sf/St}) \times 100 \leq 42.0 \quad (3),$$

wherein Sf and St are determined by following steps (a) to (c):

(a) p (p is an integer of 2 or more) thermocouples are arranged at regular intervals throughout the gas flow direction of the two or more catalyst layers provided in the reaction tube, and spot temperatures $T1_j$ (°C) (j is 1 to p) at p measurement points in the reaction tube when producing the unsaturated aldehyde at the reaction bath temperature A (°C) are obtained by using the thermocouples;
(b) for each of the measurement points, a spot heat generation temperature $T2_j$, which is a value ($T1_j$ - A) obtained by subtracting the reaction bath temperature A (°C) from the spot temperature $T1_j$ (°C), is calculated, provided that if $T1_j$ - A is a negative value, the spot heat generation temperature $T2_j$ of the relevant measurement point is set to 0; and
(c) St is defined as a value of the sum of the spot heat generation temperatures $T2_j$ at all the measurement points in the reaction tube. Sf is defined as a value of the sum of the spot heat generation temperatures $T2_k$ at the measurement points provided in the catalyst layer closest to an outlet, where k is 1 to q, and q is the number of the measurement points provided in the catalyst layer closest to the outlet, q < p.

ADVANTAGEOUS EFFECTS OF INVENTION

According to the present invention, when using an alkene or an alcohol that may produce alkenes through the intramolecular dehydration reaction as a raw material to produce a corresponding unsaturated aldehyde, it is possible to safely and stably maintain a high yield for a long period of time even in an industrial plant.

BRIEF DESCRIPTION OF DRAWINGS

FIGURE is a schematic view of an example of a production device of an unsaturated aldehyde.

DETAILED DESCRIPTION

FIGURE is a schematic view of an example of a production device 1 of an unsaturated aldehyde. As shown in FIGURE, the production device 1 includes a fixed-bed multitubular reactor having multiple reaction tubes 20 and a reaction bath 30. Inside the reaction tube 20 are provided catalyst layers 21, 22, and 23, and different catalysts are arranged in each of the catalyst layer. In addition, the temperature of the reaction tube 20 can be adjusted by the reaction bath 30. With the production device 1, a reaction product containing an unsaturated aldehyde can be obtained by, for example, introducing a reaction raw material gas containing an alkene from the top of the production device 1, and passing the raw material gas through the reaction tube 20 to react.

[Relationship between A, A1, and A2]

The present invention relates to a production method and production device of an unsaturated aldehyde, and in essence, to a method for packing catalyst layers. Here, the catalyst is packed so that, assuming the reaction bath temperature at which a yield of the unsaturated aldehyde is the highest is A (°C), and the reaction bath temperatures at which the yield is 1.0% lower than the highest value are A1 (°C) and A2 (°C), the aforementioned formulae (1) and (2). As used herein, the yield means the molar yield. In this specification, A, A1, and A2 are defined as follows.

A: the reaction bath temperature at which the yield of the unsaturated aldehyde is the highest

A1 and A2: the reaction bath temperatures at which the yield is 1.0% lower than the yield of the unsaturated aldehyde at the reaction bath temperature A

A is higher than A1, A2 is higher A, and (A2 - A1) is 10°C or higher. (A2 - A1) is more preferably 10°C or higher and 30°C or lower, even more preferably 10°C or higher and 25°C or lower, and most preferably 10°C or higher and 20°C or lower. (A2 - A1) represents a reaction bath temperature zone in which the yield of acrolein is stable (operation window), and the larger (A2 - A1) is, the higher the yield of the unsaturated aldehyde can be obtained in a wider reaction bath temperature range, which is desirable. The maximum value of the yield (the yield at the reaction bath temperature A) is, for example, 50% or more, and preferably 70% or more. Note that A1 and A2 do not have to be calculated from the relationship between the actually measured yield of acrolein and BT, but may be calculated by interpolation or extrapolation of a series of data obtained by measuring the yields of acrolein while changing BT. The reason why the yield of acrolein decreases at a reaction bath temperature lower than A (°C) is a decrease of a conversion rate of propylene as a raw material, and the reason why the yield of acrolein decreases at a reaction bath temperature higher than A (°C) is generation of carbon dioxide, acrylic acid, and the like which is typified by a successive oxidation reaction of acrolein. When the target product is acrylic acid, the catalyst according to the present invention is used in a first stage of a two-stage oxidation reaction (propylene → acrolein → acrylic acid). In this case, what is important is the total amount of acrolein and acrylic acid, so even if the reaction bath temperature becomes high, a yield of acrylic acid does not drop steeply. Therefore, when the target product is acrylic acid, the reaction bath temperature zone in which the yield is stable tends to be relatively wide. In contrast, when the target product is acrolein, the operation window tends to be relatively narrow and thus is difficult to control appropriately. As described above, the control method therefor has not been known even to those skilled in the art.
When the catalyst is packed to satisfy the formula (1) and the formula (2), the hot spot temperature (PTf, unit: °C) of the most active catalyst on the outlet side can change gently with respect to the reaction bath temperature (BT, unit: °C), so that safe and stable plant operation with a higher yield can be realized. In addition, suppression of runaway reactions will prevent thermal stress on the catalyst so that the longer lifespan is also expected. More specifically, a thermal runaway and the resulting damage or explosion of the reaction tubes can be prevented, the thermal runaway being caused by a steep change in the hot spot on the most active catalyst on the outlet side due to a slight change of the reaction bath temperature or a difference in the reaction bath temperature between multiple reaction tubes. In addition, the followings can be suppressed: a decrease in a yield of acrolein due to a cool flame reaction (cool flame) of acrolein which is caused by the fact that a large amount of heat is generated at the outlet side of the reactor and makes the gas temperature high at the outlet of the reactor; deposit of carbonaceous precipitates on the outlet side of the reaction tubes due to an autoxidation reaction of acrolein (coking or fouling); and blockage inside the reaction tubes and deterioration of the catalyst due to the latter.

[Relationship between Sf and St]

In the present invention, it is preferable that two or more catalyst layers are provided in the gas flow direction of the reaction tubes, and Sf and St related to the spot heat generation temperature satisfy the aforementioned formula (3). Sf and St are values determined by following steps (a) to (c):

(a) p (p is an integer of 2 or more) thermocouples are arranged at regular intervals throughout the gas flow direction of the two or more catalyst layers provided in the reaction tube, and spot temperatures $T1_j$ (°C) (i is 1 to p) at p measurement points in the reaction tube when producing the unsaturated aldehyde at the reaction bath temperature A (°C) are obtained by using the thermocouples;
(b) for each of the measurement points, a spot heat generation temperature $T2_j$, which is a value ($T1_j$ - A) obtained by subtracting the reaction bath temperature A (°C) from the spot temperature $T1_j$ (°C), is calculated. Note that if $T1_j$ - A is a negative value, the spot heat generation temperature $T2_j$ of the relevant measurement point is set to 0; and
(c) St is defined as a value of the sum of the spot heat generation temperature $T2_j$ at all the measurement points in the reaction tube. Sf is defined as a value of the sum of spot heat generation temperature $T2_k$ at the measurement points provided in the catalyst layer closest to the outlet (k is 1 to q, and q is the number of the measurement points provided in the catalyst layer closest to the outlet, q < p). In other words, St and Sf are defined by a formula (5) and a formula (6), respectively.

[Math. 1]

$$St = \sum_{i=1}^{p} T2_i \qquad (5)$$

$$Sf = \sum_{j=1}^{q} T2_j \qquad (6)$$

In a reactor in which the thickness of a partition wall is constant from an inlet to an outlet of a reaction tube like a multitubular reactor, according to the Fourier's law of heat conduction, Sf means the total amount of heat generated by a catalyst layer closest to the outlet, and St means the total amount of heat generated by all catalyst layers. Therefore, Sf and St satisfying the relationship of the formula (3) means that the amount of heat generated in the catalyst layer closest to the outlet is 42.0% or less of the amount of heat generated in all catalyst layers, the raw material gas certainly reacts before reaching the highly active catalyst layer closest to the outlet. It is well known to those skilled in the art that a reaction rate of partial oxidation of an alkene to an unsaturated aldehyde is first order relative to a partial pressure of the alkene. In other words, the reaction rate and reactivity (obtained by combining the reaction rate with effects of a shape of a catalyst and a dilution ratio by an inert carrier) are lower in the lower layers in the reactor. Therefore, when multi-layer packing is adopted in this reaction, it is common to select a highly active catalyst for a side closest to the outlet. However, in designing such a packing method, the degree to which the alkene is reacted before the catalyst closest to the outlet and the setting of the activity of the catalyst closest to the outlet in order to obtain the effects of the present invention have been not obvious to those skilled in the art. Note that, regarding the more preferred range of Sf/St×100, the lower limit is 1, 5, 7, 9, 11, 12, 13, 14, 15, 16, 17, or 18 in the reverse order of preference, and the upper limit is 40, 37, 35, 33, 31, 30, 29, or 28 in the reverse order of preference. In other words, Sf/St×100 is preferably 1 or more and 40 or less, more preferably 5 or more and 40 or less, more preferably 7 or more and 40 or less, more preferably 11 or more and 40 or less, more preferably 12 or more and 37 or less, more preferably 13 or more and 35 or less, more preferably 14 or more and 33 or less, more preferably 15 or more and 31 or less, more preferably 16 or more and 30 or less, more preferably 17 or more and 29 or less, and most preferably 18 or more and 28 or less.

[Measurement of Temperature Distribution inside Reaction Tube]

A thermocouple is inserted into the reactor tube at regular intervals in a depth direction in order to obtain temperature information of the catalyst layers into which the catalyst is packed and/or inert layers into which an inert carrier is packed. The way to insert the thermocouple is not limited as long as it is known to those skilled in the art, and examples thereof include the following. The direction in which the thermocouple is inserted is a depth direction of the reactor tube and/or a direction perpendicular to the depth direction of the reactor tube. The thermocouple is inserted directly into the reactor tube in parallel with the reactor tube, and/or a case (thermowell) into which the thermocouple will be inserted is inserted into the reactor, and then the thermocouple is inserted into the case (that is, the reactor tube has a double-tube structure). The way to move the thermocouple over time is a fixed type in which it does not move at all; and/or a type in which it moves to an arbitrary position within the reactor tube over time.

Note that the thermocouple is inserted into not all but some of the reaction tubes, and typically 5 or more and 100 or less, preferably 6 or more and 50 or less, more preferably 7 or more and 40 or less, or particularly preferably 8 or more and 16 or less reactor tubes are selected as the targets for the insertion out of thousands to tens of thousands of reaction tubes. In addition, regarding the multitubular reactor, the reactor tubes into which the thermocouple is to be inserted may be selected from those in only some of multiple sections divided in a plane perpendicular to the flow direction of the gas, or selected as evenly as possible from those in all the sections. However, in order to grasp the temperature of the entire reactor, at least one reactor tube is preferably selected from each of the sections that are 40% or more, more preferably 60% or more, and even more preferably 75% or more of the total sections. Note that in present invention, unless otherwise specified, a reaction tube into which a thermocouple is inserted is also simply referred to as a "reactor tube".

Furthermore, with respect to the positions of the thermocouples in the depth direction, a method for arranging the thermocouples at equal intervals, or a method for changing the intervals of the thermocouples as necessary may be adopted, but the method is not particularly limited thereto. When the thermocouples are arranged at equal interval, it is preferable to obtain temperature measurement data at intervals of 10 cm or less in order to accurately determine Sf and St. When the intervals of the thermocouples are changed, in particular in exothermic reactions in the present invention, since the temperature distribution exhibits a steep rise in the depth direction in the catalyst-packed layer on the gas inlet side, a method is preferable in which the intervals of the positions of the thermocouples are narrower on the gas inlet side because the temperature distribution exhibits a steep rise in the depth direction in the catalyst-packed layer on the gas inlet side, and the intervals of the positions of the thermocouples are wider in the catalyst-packed layer on the gas outlet side. Furthermore, it is preferable to shift the measurement position in each reaction tube in the depth direction rather than measuring the temperature information at the same position in the depth direction for all of the plurality of selected reactor tubes. This is because it becomes easier to grasp the temperature distribution throughout the reactor.

By setting the section and number of the reaction tubes into which the thermocouples are inserted, and the depth of the measurement points as described above, the temperature distribution inside the reaction tubes can be acquired efficiently with a small number of the thermocouples.

[Catalyst]

The catalyst used in the present invention is preferably a catalyst having composition represented by the following formula (I):

$$Mo_aBi_bNi_cCo_dFe_eX_fY_gZ_hO_i \qquad (I)$$

In the formula (I) above:

Mo, Bi, Ni, Co, and Fe represent molybdenum, bismuth, nickel, cobalt, and iron, respectively;
X represents at least one element selected from tungsten, antimony, tin, zinc, chromium, manganese, magnesium, silicon, aluminum, cerium, and titanium;
Y represents at least one element selected from sodium, potassium, cesium, rubidium, and thallium;
Z represents at least one element belonging to groups 1 to 16 on the periodic table and selected from elements other than Mo, Bi, Ni, Co, Fe, X, Y, and O described above; and
a, b, c, d, e, f, g, h, and i represent the number of atoms of molybdenum, bismuth, nickel, cobalt, iron, X, Y, Z, and oxygen, respectively, wherein, when $a = 12$, $0 < b \leq 7.0$, $0 \leq c \leq 10$, $0 < d \leq 10$, $0 < c + d \leq 20$, $0 < e \leq 5.0$, $0 \leq f \leq 2.0$, $0 < g \leq 3.0$, and $0 \leq h \leq 5.0$ are satisfied, and i is a value determined by an oxidation state of each element.

In the formula (I) above, preferred ranges of b to h when $a = 12$ is as follows.
For b, the lower limit is 0.10, 0.20, 0.30, 0.40, 0.50, 0.60, or 0.70 in the reverse order of preference, and the upper limit is 6.0, 5.0, 4.0, 3.0, 2.0, 1.8, 1.5, 1.2, or 1.0 in the reverse order of preference. In other words, b is preferably 0.10 or more and 6.0 or less, more preferably 0.10 or more and 5.0 or less, more preferably 0.10 or more and 4.0 or less, more preferably 0.20 or more and 3.0 or less, more preferably 0.30 or more and 2.0 or less, more preferably 0.40 or more and 1.8 or less, more preferably 0.50 or more and 1.5 or less, more preferably 0.60 or more and 1.2 or less, and most preferably 0.70 or more and 1.0 or less.

For c, the lower limit is 0.20, 0.50, 0.80, 1.0, 1.5, 1.8, 2.0, 2.5, or 2.8 in the reverse order of preference, and the upper limit is 8.0, 7.0, 6.0, 5.0, 4.0, 3.5, or 3.3 in the reverse order of preference. In other words, c is preferably 0.20 or more and 8.0 or less, more preferably 0.50 or more and 8.0 or less, more preferably 0.80 or more and 8.0 or less, more preferably 1.0 or more and 7.0 or less, more preferably 1.5 or more and 6.0 or less, more preferably 1.8 or more and 5.0 or less, more preferably 2.0 or more and 4.0 or less, more preferably 2.5 or more and 3.5 or less, and most preferably 2.8 or more and 3.3 or less.

For d, the lower limit is 1.0, 2.0, 3.0, 4.0, or 5.0 in the reverse order of preference, and the upper limit is 9.5, 9.0, 8.5, 8.0, 7.5, 7.0, 6.5, or 6.3 in the reverse order of preference. In other words, d is preferably 1.0 or more and 9.5 or less, more preferably 1.0 or more and 9.0 or less, more preferably 1.0 or more and 8.5 or less, more preferably 1.0 or more and 8.0 or less, more preferably 2.0 or more and 7.5 or less, more preferably 3.0 or more and 7.0 or less, more preferably 4.0 or more and 6.3 or less, and most preferably 5.0 or more and 6.3 or less.

For c + d, the lower limit is 0.0, 2.0, 4.0, 6.0, 8.0, or 8.3 in the reverse order of preference, and the upper limit is 20.0, 15.0, 12.5, 11.0, 10.0, or 9.0 in the reverse order of preference. In other words, c + d is preferably 0.0 or more and 20.0 or less, more preferably 2.0 or more and 15.0 or less, more preferably 4.0 or more and 12.5 or less, more preferably 6.0 or more and 11.0 or less, more preferably 8.0 or more and 10.0 or less, and most preferably 8.3 or more and 9.0 or less.

For e, the lower limit is 0.10, 0.20, 0.50, 0.80, 1.0, 1.5, or 1.6 in the reverse order of preference, and the upper limit is 4.5, 4.0, 3.5, 3.0, 2.5, 2.0, or 1.9 in the reverse order of preference. In other words, e is preferably 0.10 or more and 4.5 or less, more preferably 0.20 or more and 4.0 or less, more preferably 0.50 or more and 3.5 or less, more preferably 0.80 or more and 3.0 or less, more preferably 1.0 or more and 2.5 or less, more preferably 1.5 or more and 2.0 or less, and most preferably 1.6 or more and 1.9 or less.

For f, the upper limit is 1.8, 1.5, 1.0, 0.80, or 0.50 in the reverse order of preference, and the lower limit is preferably 0. In other words, f is preferably 0 or more and 1.8 or less, more preferably 0 or more and 1.5 or less, more preferably 0 or more and 1.0 or less, more preferably 0 or more and 0.80 or less, more preferably 0 or more and 0.50 or less, and most preferably 0.

For g, the lower limit is 0.010, 0.020, 0.030, 0.040, 0.050, or 0.060 in the reverse order of preference, and the upper limit is 2.0, 1.0, 0.50, 0.40, 0.30, 0.20, 0.15, or 0.090 in the reverse order of preference. In other words, g is preferably 0.010 or more and 2.0 or less, more preferably 0.010 or more and 1.0 or less, more preferably 0.010 or more and 0.50 or less, more preferably 0.020 or more and 0.40 or less, more preferably 0.030 or more and 0.30 or less, more preferably 0.040 or more and 0.20 or less, more preferably 0.050 or more and 0.15 or less, and most preferably 0.060 or more and 0.090 or less.

For h, the upper limit is 4.0, 3.0, 2.0, 1.8, 1.5, 1.0, 0.80, or 0.50 in the reverse order of preference, and the lower limit is preferably 0. In other words, h is preferably 0 or more and 4.0 or less, more preferably 0 or more and 3.0 or less, more preferably 0 or more and 2.0 or less, more preferably 0 or more and 1.8 or less, more preferably 0 or more and 1.5 or less, more preferably 0 or more and 1.0 or less, more preferably 0 or more and 0.80 or less, more preferably 0 or more and 0.50 or less, and most preferably 0.

X in the formula (1) is preferably tungsten, antimony, zinc, magnesium, or cerium, and particularly preferably antimony or zinc.

Y in the formula (1) is preferably sodium, potassium, or cerium, and particularly preferably potassium or cesium.

Z in the formula (1) is preferably vanadium, copper, niobium, zirconium, calcium, beryllium, strontium, barium, lead, or phosphorus.

[Catalyst in First Layer]

In the present invention, it is preferable that a catalytically active component contained in a first layer (a catalyst layer closest to the inlet of the reaction raw material gas) has composition represented by the following formula (I-1). Note that, when three or more catalyst layers are provided, it is preferable that all catalyst layers except the catalyst layer closest to the outlet contain a catalyst having the composition represented by the formula (I-1).

$$Mo_{a1}Bi_{b1}Ni_{c1}CO_{d1}Fe_{e1}X_{f1}Cs_{g1}Z_{h1}O_{i1} \qquad (I-1)$$

In the formula (I-1):

Mo, Bi, Ni, Co, Fe, Cs, and O represent molybdenum, bismuth, nickel, cobalt, iron, cesium, and oxygen, respectively;
X represents at least one element selected from tungsten, antimony, tin, zinc, chromium, manganese, magnesium, silicon, aluminum, cerium, and titanium;

Z represents at least one element belonging to groups 1 to 16 on the periodic table and selected from elements other than Mo, Bi, Ni, Co, Fe, Cs, O, and X described above; and

a1, b1, c1, d1, e1, f1, g1, h1, and i1 represent the number of atoms of molybdenum, bismuth, nickel, cobalt, iron, X, Cs, Z, and oxygen, respectively, wherein, when a1 = 12, $0 < b1 \leq 7.0$, $0 \leq c1 \leq 10$, $0 < d1 \leq 10$, $0 < e1 \leq 5.0$, $0 \leq f1 \leq 2.0$, $0 < g1 \leq 3.0$, and $0 \leq h1 \leq 5.0$ are satisfied, and i1 is a value determined by an oxidation state of each element.

b1 to f1 and h1, as well as X and Z when a1 = 12 in the formula (I-1) are the same as b to f and h, as well as X and Z in the formula (I), including preferred aspects thereof.

For g1, the lower limit is 0.0010, 0.0050, 0.010, 0.015, 0.020, or 0.030 in the reverse order of preference, and the upper limit is 2.0, 1.0, 0.50, 0.40, 0.30, 0.20, 0.15, 0.090, or 0.060 in the reverse order of preference. In other words, g1 is preferably 0.0010 or more and 2.0 or less, more preferably 0.0010 or more and 1.0 or less, more preferably 0.0010 or more and 0.50 or less, more preferably 0.0010 or more and 0.40 or less, more preferably 0.0050 or more and 0.30 or less, more preferably 0.010 or more and 0.20 or less, more preferably 0.015 or more and 0.15 or less, more preferably 0.020 or more and 0.090 or less, and most preferably 0.030 or more and 0.060 or less.

[Catalyst Closest to Outlet]

In the present invention, it is preferable that a catalytically active component contained in a catalyst layer closest to the outlet has composition represented by the following formula (I-2). When three catalyst layers are provided, for example, it is preferable that a catalytically active component contained in a third catalyst layer has the composition represented by the formula (I-2). In case of multi-layer packing with four or more catalyst layers, it is also preferable that a catalytically active component contained in a catalyst layer closest to the outlet has the composition represented by the formula (I-2).

$$Mo_{a2}Bi_{b2}Ni_{c2}Co_{d2}Fe_{e2}X_{f2}K_{g2}Z_{h2}O_{i2} \qquad (I-2)$$

In the formula:

Mo, Bi, Ni, Co, Fe, K, and O represent molybdenum, bismuth, nickel, cobalt, iron, potassium, and oxygen, respectively;

X represents at least one element selected from tungsten, antimony, tin, zinc, chromium, manganese, magnesium, silicon, aluminum, cerium, and titanium;

Z represents at least one element belonging to groups 1 to 16 on the periodic table and selected from elements other than Mo, Bi, Ni, Co, Fe, K, O, and X described above; and

a2, b2, c2, d2, e2, f2, g2, h2, and i2 represent the number of atoms of molybdenum, bismuth, nickel, cobalt, iron, X, K, Z, and oxygen, respectively, wherein, when a2 = 12, $0 < b2 \leq 7.0$, $0 \leq c2 \leq 10$, $0 < d2 \leq 10$, $0 < e2 \leq 5.0$, $0 \leq f2 \leq 2.0$, $0 \leq g2 \leq 3.0$, and $0 \leq h2 \leq 5.0$ are satisfied, and i2 is a value determined by an oxidation state of each element.

b2 to f2, h2, X, and Z when a2 = 12 in the formula (I-2) are the same as b to f, h, X, and Z in the formula (I), including preferred aspects thereof.

For g2, the lower limit is 0.0010, 0.0050, 0.010, 0.015, 0.020, or 0.030 in the reverse order of preference, and the upper limit is 2.0, 1.0, 0.50, 0.40, 0.30, 0.20, 0.15, or 0.10 in the reverse order of preference. In other words, g2 is preferably 0.0010 or more and 2.0 or less, more preferably 0.0010 or more and 1.0 or less, more preferably 0.0010 or more and 0.50 or less, more preferably 0.0050 or more and 0.40 or less, more preferably 0.010 or more and 0.30 or less, more preferably 0.015 or more and 0.20 or less, more preferably 0.020 or more and 0.15 or less, and most preferably 0.030 or more and 0.10 or less.

[Packing Method]

As a method for adjusting the relationship between A, A1, and A2, and a method for adjusting the relationship between St and Sf, various methods including the followings can be considered and used alone or in combination:

(1) a method for controlling the relative activity of each catalyst layer;
(2) a method for controlling the particle size in each catalyst layer;
(3) a method for providing an inert material layer on the outlet side of the reaction tube; and
(4) a method for setting the gas temperature at the inlet side of the reaction tube high.

In the method (1) for controlling the relative activity of each catalyst layer, the catalyst is packed into a differential reactor, and a raw material gas conversion ratio x is obtained at a specific BT, which is used for calculating a reaction rate according to an integral reaction rate equation. For example, in the oxidation reaction of propylene, since it can be expressed by a first-order rate equation relative to a propylene concentration, a reaction rate k of this reaction is calculated according to the following formula (II).

$$k = -\mathrm{Ln}\,(1 - x/100) \quad (II)$$

Here, k is the reaction rate (unitless) of this reaction, Ln is the natural logarithm, and x is the propylene conversion ratio (unit: %).

Next, after calculating the reaction rate of each catalyst layer under the same condition using this method, an actual reaction rate ak of each catalyst layer in the plant is calculated according to the following formula (III).

$$ak = \text{(dilution ratio of relevant catalyst layer)} \times \text{(packing length of relevant catalyst layer)} \times \text{(reaction rate k of catalyst in relevant catalyst layer)} \quad (III)$$

Here, the dilution ratio means a dilution ratio by an inert material described below, and the packing length refers to a length of the packed catalyst layer expressed in cm. Note that it is preferable to use an actually measured value rather than a designed value for the packing length. Since there are often multiple reaction tubes in a plant, an average value obtained from the measurement results of some of them can also be used. For example, the catalysts are successively packed into the reaction tube from the top of the reaction tube to the bottom, and the packing lengths can be calculated by measuring a length of a space above each layer using a tape measure or the like.

ak of each layer thus calculated is used for calculating akt/akn according to the following formula (IV). Regarding a preferred range of akt/akn, the lower limit is 1.41, 1.42, 1.43, 1.44, or 1.45 in the reverse order of preference, and the upper limit is 10.00, 7.50, 5.00, 4.00, 3.00, 2.75, 2.65, 2.55, or 2.50 in the reverse order of preference. In other words, akt/akn is preferably 1.41 or more and 10.00 or less, more preferably 1.41 or more and 7.50 or less, more preferably 1.41 or more and 5.00 or less, more preferably 1.41 or more and 4.00 or less, more preferably 1.41 or more and 3.00 or less, more preferably 1.42 or more and 2.75 or less, more preferably 1.42 or more and 2.65 or less, more preferably 1.42 or more and 2.55 or less, and most preferably 1.45 or more and 2.50 or less.

$$akt/akn = \text{(value of sum of actual reaction rates ak of all catalyst layers)}/\text{(actual reaction rate ak of catalyst layer closest to outlet of reaction tube)} \quad (IV)$$

Note that, in the present invention, the raw material gas conversion ratio x described above is calculated according to the method described below. Four grams of a catalyst is packed into a reaction tube with an inner diameter of 28.4 mm, with the catalyst diluted with an inert material for preventing the occurrence of hot spots. Then, a reaction is carried out at a molar ratio of propylene:oxygen:nitrogen:water = 1:1.7:6.4:3.0, and at a reaction bath temperature of 360°C such that space velocity (GHSV) of propylene is 400hr$^{-1}$. A propylene flow rate at an outlet is calculated by calibrated gas chromatography, and the propylene conversion ratio x is calculated according to the following formula.

$$x = 100 - \text{(propylene flow rate at outlet)} / \text{(propylene flow rate at inlet)} \times 100$$

In the method (2) controlling the particle size in each catalyst layer, a catalyst particle size in a catalyst layer closest to the outlet of the reaction tube is only required to be different from and may be larger or smaller than catalyst particle sizes in other catalyst layers. When the particle size of the catalyst on the outlet side of the reaction tube is larger, the retention of gas on the outlet side is reduced, resulting in an increase in the yield of the target product, and as a secondary effect, the operation window can be adjusted to be wider. When the particle size of the catalyst on the outlet side of the reaction tube is smaller, the pressure and reaction rate increase on the inlet side, and the reaction proceeds steadily on the inlet side, so that the operation window can be adjusted to be wider. Since these effects vary depending on a pressure setting inside the reaction tube, the reaction bath temperature, the inner diameter of the reaction tube, a molar ratio of the gas at the inlet, and propylene concentration, the particle size in each catalyst layer should be appropriately controlled according to each condition. For example, when the pressure in the reaction tube is high, and/or the reaction bath temperature is low, and/or the inner diameter of the reaction tube is large, and/or the inlet propylene concentration is low, it is preferable to increase the particle size in each catalyst layer, and it is particularly preferable to increase the particle size in the catalyst layer on the inlet side of the reaction tube. Regarding a preferred range of a particle size ratio ((the catalyst particle size in the catalyst layer closest to the outlet of the reaction tube) / (the catalyst particle size in other catalyst layers)), the lower limit is 0.50, 0.60, 0.70, 0.80, 0.90, or 1.00 in the reverse order of preference, and the upper limit is 1.50, 1.40, 1.30, 1.20, or 1.10 in the reverse order of preference. In other words, the particle size ratio is preferably 0.50 or more and 1.50 or less, more preferably 0.60 or more and 1.50 or less, more preferably 0.70 or more and 1.40 or less, more preferably 0.80 or more and 1.30 or less, more preferably 0.90 or more and 1.20 or less, and most preferably 1.00 or more and 1.10 or less. When three or more catalyst layers are provided, a weighted average of the catalyst particle sizes weighted by the packing length of each catalyst layer other than the catalyst layer closest to the outlet is defined as a "catalyst particle size of the other catalyst layers".

In the method (3) for providing the inert material layer on the outlet side of the reaction tube, the pressure on the inlet side of the reaction tube particularly increases by providing an inert material layer closer to the outlet than the catalyst layer, so that the reaction can proceed steadily on the inlet side of the reaction tube. A packing length of the inert material on the outlet side varies depending on the raw material load and the diameter of the reaction tube, and is, for example, 5 cm or more, preferably 10 cm or more, more preferably 20 cm or more, and most preferably 30 cm or more. The packing length of the inert material on the outlet side may be 50 cm or less, for example.

In the method (4) for setting the gas temperature at the inlet side of the reaction tube high, the catalyst is easily activated on the inlet side of the reaction tube by the increased inlet gas temperature, so that the reaction can proceed steadily. The inlet gas temperature is 150°C or higher, 200°C or higher, 250°C or higher, 270°C or higher, 290°C or higher, 300°C or higher, 310°C or higher, 320°C or higher, 330°C or higher, or 340°C or higher in the reverse order of preference. The inlet gas temperature is 360°C or lower, for example.

[Packing Length of Catalyst Layer]

A method for adjusting the packing length of the catalyst layer in the aforementioned method (1) will be described in detail with the packing length of the catalyst layer defined as follows.

Ln: packing length of nth layer counted from gas inlet side of reaction tube when n catalyst layers are provided in gas flow direction of reaction tube
L: the sum of packing lengths of first to n-1 layers counted from gas inlet side of reaction tube

In a preferred packing method, L/Ln is 1.5 or more and 3.5 or less. In addition, it is preferable that the catalyst layers have the aforementioned catalyst composition. Regarding L/Ln, the upper limit is more preferably 3.4, 3.3, 3.2, or 3.1, and particularly preferably 3.0. The lower limit is preferably 1.6, 1.7, 1.8, or 1.9, and particularly preferably 2.0. Therefore, L/Ln is 1.6 or more and 3.4 or less, more preferably 1.7 or more and 3.3 or less, more preferably 1.8 or more and 3.2 or less, more preferably 1.9 or more and 3.1 or less, and most preferably 2.0 or more and 3.0 or less.

The number n of the catalyst layers is preferably 2 to 5, more preferably 2 to 4, particularly preferably 2 to 3, and most preferably 3.

The shape of the catalyst contained in the catalyst layer used in the present invention is not particularly limited, and a spherical shape, cylindrical shape, ring shape, powder shape or the like can be used. In particular, the spherical shape is preferred.

Note that, in case of two-layer packing, both the catalyst contained in the upper layer and the catalyst contained in the lower layer may be diluted with an inert material, but a method in which neither the upper layer nor the lower layer is diluted is preferable.

A preferred range of the dilution ratio by the inert material is described below. As used herein, the dilution ratio is a value representing a mass ratio occupied by the catalyst in the catalyst layer comprising the catalyst and the inert material. For example, a description that the catalyst layer is in 80% by mass dilution means that the catalyst accounts for 80% by mass and the inert material accounts for 20% by mass. Note that, when a catalytically active component is loaded on an inert carrier to form a catalyst as in the method for manufacturing a catalyst described below, the dilution ratio is calculated based on the mass of the catalyst including the inert carrier. A preferred embodiment will be described using the case where n = 2 or 3 as an example, but the present invention is not limited thereto.

The preferred aspects of the method for packing the catalyst layers are the following 1) and 2).

1) n = 2. The upper layer is a catalyst containing a catalytically active component represented by the formula (I-1) and has a dilution ratio of 100% by mass. The lower layer is a catalyst containing a catalytically active component represented by the formula (I-2) and has a dilution ratio of 100% by mass.
2) n = 3. The middle layer is a catalyst containing a catalytically active component represented by the formula (I-1) and has a dilution ratio of 100% by mass. The upper layer is a catalyst obtained by diluting the same catalyst as in the middle layer with an inert material, and has a dilution ratio of 60% by mass or more and less than 100% by mass. The lower layer is a catalyst containing a catalytically active component represented by the formula (I-2) and has a dilution ratio of 100% by mass.

There is also a method in which an inert material layer (inert layer) is provided closer to the inlet of the reaction tube than the catalyst layer, and this method may be adopted as long as it does not impair the effects of the present invention. However, in the present invention, it is preferable not to have an inert layer closer to the inlet side of the reaction tube than the catalyst layer.

Examples of the inert material include known materials such as silica, alumina, titania, zirconia, niobia, silica-alumina, silicon carbide, carbides, and mixtures thereof. Among them, silica, alumina, or a mixture thereof are preferred, silica, or alumina is particularly preferred, and the mixture of silica and alumina is most preferred.

The shape of the inert material is not particularly limited, but is preferably spherical. The average particle size of the inert material is preferably 3 mm to 10 mm, more preferably 3.5 mm to 9 mm, and particularly preferably 4 mm to 8 mm.

[Method for Manufacturing Catalyst]

The catalyst used in the present invention can be manufactured by the following steps a) to e), for example.

<Step a) Preparation>

Generally, a starting material of each element composing the catalytically active component is not particularly limited. As a raw material for a molybdenum components, the followings can be used: molybdenum oxides such as molybdenum trioxide; molybdic acids or salts thereof such as molybdic acids and ammonium molybdates; heteropoly acids containing molybdenum such as phosphomolybdic acid and silicomolybdic acid, or salts thereof. Preferred is a case where ammonium molybdate is used, resulting in a high-performance catalyst. Ammonium molybdate encompasses a plurality of compounds such as ammonium dimolybdate, ammonium tetramolybdate, and ammonium heptamolybdate, and among them, a case where ammonium heptamolybdate is used is most preferred.

As a raw material for a bismuth component, the followings can be used: bismuth salts such as bismuth nitrate, bismuth subcarbonate, bismuth sulfate, and bismuth acetate; bismuth trioxide; and metallic bismuth. Preferred is bismuth nitrate, resulting in a high-performance catalyst. As raw materials for iron, cobalt, nickel, and other elements, oxides are typically used, or alternatively, nitrates, carbonates, organic acid salts, hydroxides, and the like that can be converted to oxides by ignition, or mixtures thereof can be used. For example, an aqueous solution or a slurry containing catalyst components is obtained by: dissolving and mixing a raw material for an iron component and a raw material for a cobalt and/or nickel component in water at a desired ratio under a condition of 10 to 80°C; mixing with a separately prepared aqueous solution or slurry of a raw material for a molybdenum component and a raw material for a Z component under a condition of 20 to 90°C; stirring while heating for 1 hour under a condition of 20 to 90°C; and then adding a aqueous solution in which a raw material for a bismuth component is dissolved, and if necessary, a raw material for an X component and a raw material for a Y component. Hereinafter, the aqueous solution or the slurry thus obtained is collectively referred to as a preparation solution (A).

Here, the preparation solution (A) does not necessarily contain all the constituent elements of the catalytically active component, and some of the elements or some of the amount of the elements may be added in a subsequent step. In addition, if the amount of water in which the raw material for each component is dissolved for preparing the preparation solution (A) is not suitable for the preparation, and if a concentration of an acid in the aqueous solution sufficient to dissolve the raw materials is not suitable for the preparation within a range of, for example, 5% by mass~99% by mass when an acid such as sulfuric acid, nitric acid, hydrochloric acid, tartaric acid, or acetic acid is added for dissolution, the preparation solution (A) may take a form of a clay-like mass. In this case, an excellent catalyst cannot be obtained. The preparation solution (A) is preferably in a form of an aqueous solution or a slurry, since an excellent catalyst can be obtained.

<Step b) Drying>

Then, the preparation solution (A) obtained above is dried to obtain a dry powder. There is no particular limitation on the drying method as long as it can completely dry the preparation solution (A), and examples include drum drying, freeze drying, spray drying, and evaporation to dryness. Among them, spray drying is particularly preferred in the present invention, which can dry the slurry into a powder or granules in a short time. A drying temperature for spray drying varies depending on a concentration, a delivery speed, or the like of the slurry, and generally, a temperature at an outlet of a dryer is 70 to 150°C. It is also preferable to dry the slurry so that an average particle size of the resulting dry powder is 10 to 700 $\mu$m. In this way, a dry powder (B) is obtained.

<Step c) Pre-calcination>

The resulting dry powder (B) is calcined under air flow at 200°C to 600°C, preferably 300°C to 600°C, which tends to improve moldability, mechanical strength, and catalytic performance of the catalyst. Calcination time is preferably 1 to 12 hours. In this way, a pre-calcined powder (C) is obtained.

<Step d) Molding>

Although there is no particular limitation on the molding method, a method using a tableting machine, an extrusion machine, or the like is preferred when molding into a cylindrical or ring shape. Molding into a spherical shape is more preferred, and in this case, the pre-calcined powder (C) may be molded into a spherical shape using a molding machine, but a method is preferred in which the pre-calcined powder (C) (containing a molding aid or a strength improver, if necessary) is loaded on a carrier such as an inert ceramic. Here, as a loading method, the rolling granulation method, the method using a centrifugal fluidized coating device, the wash coat method, and the like are widely known. There is no particular limitation on the method as long as the pre-calcined powder (C) can be uniformly loaded on the carrier, but when considering the production efficiency of the catalyst and the performance of the prepared catalyst, a method is preferred in which: in a device having a planar or uneven disk at the bottom of a fixed cylindrical container, the disk is rotated at high speed so that the carrier charged in the container is vigorously stirred by repeated rotation and revolution of the carrier itself, and the pre-calcined powder (C) as well as, if necessary, the molding aid and/or the strength improver are added thereto in order to load the powder component on the carrier. In this way, a molded body (D) is obtained.

Note that it is preferable to use a binder for the loading. Specific examples of the binder that can be used include: water, ethanol, methanol, propanol, polyhydric alcohols, polyvinyl alcohol as a polymeric binder, a silica sol aqueous solution as an inorganic binder, and the like. Ethanol, methanol, propanol, and polyhydric alcohols are preferred, and diols such as ethylene glycol and triols such as glycerin are more preferred. By using an appropriate amount of a glycerin aqueous solution, moldability is improved, resulting in a high-performance catalyst with high mechanical strength. Specifically, when an aqueous solution of glycerin with a concentration of 5% by mass or more is used, a catalyst with particularly high performance can be obtained. These binders are typically used in an amount of 2 to 80 parts by mass with respect to 100 parts by mass of the pre-calcined powder (C). An inert carrier with a diameter of 2 to 8 mm is usually used, and the pre-calcined powder (C) is loaded on it. The loading ratio is determined in consideration of the use conditions of the catalyst such as space velocity of reaction raw materials and concentration of raw materials, and is typically 20% by mass to 80% by mass. Here, the loading ratio is defined by the following formula (4).

Loading ratio (% by mass) = 100 × [mass of pre-calcined powder (C) used in molding/(mass of pre- calcined powder (C) used in molding + mass of inert carrier used in molding)]  (4)

<Step e) Main Calcination>

The molded body (D) obtained in the step (d) is calcined at 200 to 600°C for 1 to 12 hours, which tends to improve catalytic activity and selectivity. The calcining temperature is preferably 400°C or higher and 600°C or lower, and more preferably 500°C or higher and 600°C or lower. Air is the preferred gas to be passed through because it is simple, but it is also possible to use inert gases, gases for creating a reducing atmosphere, and mixtures thereof. Examples of the inert gases include nitrogen and carbon dioxide. Examples of the gases for creating a reducing atmosphere include gases containing nitrogen oxides, gases containing ammonia, and a hydrogen gas. In this way, a catalyst (E) is obtained.

[Concentration of Alkene in Raw Material]

A gas-phase catalytic oxidation reaction of an alkene in the present invention is carried out by introducing a mixed gas having composition of raw material gas of 6 to 12% by volume of the an alkene (more preferably 6 to 10% by volume), 5 to 18% by volume of molecular oxygen, 0 to 60% by volume of water vapor, and 20 to 70% by volume of an inert gas such as nitrogen or carbon dioxide gas onto the catalyst prepared as described above at a temperature range of 250 to 450°C and a pressure of normal pressure to 10 atm, preferably normal pressure to 5 atm, more preferably normal pressure to 3 atm, for a contact time of 0.5 to 10 seconds.

The volume ratio of oxygen to the alkene in the raw material gas (oxygen/alkene) is preferably 1.0 or more and 1.8 or less. For oxygen/alkene, the upper limit is more preferably 1.7 or 1.6 in the reverse order of preference, and more preferably 1.5. The more preferred lower limit is 1.1, 1.2, or 1.3 in the reverse order of preference. Thus, oxygen/alkene is 1.1 or more and 1.7 or less, more preferably 1.2 or more and 1.6 or less, and most preferably 1.3 or more and 1.5 or less.

Note that the "alkene" in the present invention should include alcohols that may produce alkenes in the intramolecular dehydration reaction, such as tertiary-butyl alcohol. Higher space velocity of a reaction substrate such as the alkene with respect to a catalyst volume (reaction substrate supply rate (NL/hr)/catalyst packing space volume (L)) is preferred from the viewpoint of production efficiency. However, if it is too high, the yield of the target product may decrease and the lifespan of the catalyst may be shortened. Therefore, in practice, the space velocity of the reaction substrate with respect to the catalyst volume is preferably in a range of 40 to 200 hr$^{-1}$, and more preferably in a range of 60 to

180 hr$^{-1}$. Here, NL represents a volume of the reaction substrate under standard conditions. The conversion ratio of the alkene is preferably near the conversion ratio at which a high yield of acrolein can be obtained, and is typically 90 to 99.9%, preferably 95 to 99.5%, and more preferably 96 to 99%.

If the catalyst layer on the reaction gas outlet side is too short, the overall activity of the catalyst layer decreases, which may result in an excessive increase of the reaction bath temperature required to achieve a normal raw material conversion ratio and obtain the target product. If the reaction bath temperature is too high, hot spots become too hot, which may result in deterioration of the catalyst and degradation of performance of the catalyst. In some cases, early deterioration of the catalyst on the gas inlet side may cause high-temperature hot spots in the highly active catalyst layer on the gas outlet side, which may result in a steep drop of the selectivity and yield of the target product. Therefore, it is also necessary to consider a balance between the catalysts on the gas inlet side and the gas outlet side for preventing the decrease in the overall activity of the catalyst layer and the excessive increase of the reaction bath temperature due to a too short catalyst layer on the outlet side. The reaction bath temperature cannot be generalized because it is set appropriately depending on characteristics of the catalyst, usage conditions, a required lifespan of the catalyst, and the like, but the reaction bath temperature at the beginning of the reaction is preferably 350°C or lower, and more preferably 340°C or lower. In addition, its lower limit is 300°C or higher, and more preferably 310°C or higher. In other words, the reaction bath temperature at the beginning of the reaction is preferably 300°C or higher and 350°C or lower, and more preferably 310°C or higher and 340°C or lower. Note that the reaction bath temperature is a set temperature, which is set to obtain an appropriate raw material conversion ratio.

By carrying out the production method described above in an industrial plant, the yield of the unsaturated aldehyde can be improved and runaway at the outlet side of the highly active gas can be suppressed. This makes it possible to stably operate the industrial plant while maintaining a high yield for a long period of time. This effect is believed to be due to the fact that the occupancy of the catalyst layer with relatively high selectivity exceeds the occupancy of the catalyst layer with relatively high activity, thereby increasing the contribution of the highly selective catalyst to the reaction.

EXAMPLES

The present invention will be further described below with reference to specific examples, but the present invention should not be limited to these examples without departing from the scope of the invention.

In the following, an acrolein is defined as follows:

$$\text{Acrolein (mol\%)} = (\text{moles of acrolein produced/moles of propylene supplied}) \times 100$$

[Production Example (Preparation of Catalyst)]

(Catalyst A1)

Four hundred and twenty-three point seven parts by mass of ammonium molybdate and 0.73 parts by mass of potassium nitrate were dissolved in 3000 parts by mass of distilled water while heating and stirring in order to obtain an aqueous solution (preparation solution 1). Separately, 378.4 parts by mass of cobalt nitrate, 139.6 parts by mass of nickel nitrate, and 161.6 parts by mass of ferric nitrate were dissolved in 1000 parts by mass of distilled water in order to prepare an aqueous solution (preparation solution 2), and bismuth nitrate 97.1 parts by mass was dissolved in 200 parts by mass of distilled water acidified with 81 parts by mass of concentrated nitric acid in order to prepare an aqueous solution (preparation solution 3). The preparation solution 2 and the preparation solution 3 were successively mixed with the preparation solution 1 while vigorously stirring, and the resulted suspension is dried by using a spray dryer and calcined at 440°C for 6 hours in order to obtain a pre-calcined powder. Here, the compositional ratio of the catalytically active component excluding oxygen was Mo:Bi:Fe:Co:Ni:K = 12:1.0:2.0:6.5:3.0:0.050 in atomic ratio. Then, a powder obtained by mixing 5 parts by mass of crystalline cellulose with 100 parts by mass of the pre-calcined powder was loaded on an inert carrier (a spherical material with a diameter of 4.5 mm mainly composed of alumina and silica) while adjusting the mass of the carrier and the mass of the pre-calcined powder used for molding so as to obtain a loading ratio defined by the formula (4) above of 50% by mass. Twenty percent by mass of glycerin aqueous solution was used as a binder, and the powder was loaded and molded into a sphere with a diameter of 5.20 mm to obtain a loaded catalyst. The loaded catalyst was calcined at a calcining temperature of 530°C for 4 hours in an air atmosphere in order to obtain a catalyst A1. The activity of the catalyst A1 was evaluated as follows. Four grams of the catalyst was diluted with an inert material for preventing heat accumulation and then packed into a reaction tube with an inner diameter of 28.4 mm, and a reaction was carried out with a molar ratio of propylene:oxygen:nitrogen:water = 1:1.7:6.4:3.0 and at a reaction bath temperature of 360°C for obtaining space velocity (GHSV) of propylene = 400hr$^{-1}$. A propylene flow rate at an outlet was calculated by calibrated gas chromatography, and a propylene conversion ratio x was calculated. Based on this, a reaction rate k of the catalyst A1 calculated according to the formula (II) above was 0.63.

(Catalyst B1)

A pre-calcined powder with an atomic ratio of Mo:Bi:Fe:Co:Ni:Cs = 12:1.0:2.0:6.5:3.0:0.030 was obtained in the same manner as in the production of the catalyst A1 except that potassium nitrate in the raw material was changed to cesium nitrate. Subsequent steps were also performed in exactly the same manner as in the production of the catalyst A1 to obtain a catalyst B1 with a diameter of 5.20 mm. A reaction rate k of the catalyst B1 was 0.35.

(Catalyst B2)

A catalyst B2 with a diameter of 5.00 mm was obtained in exactly the same manner as the catalyst B1, except that the pre-calcined powder obtained during the production of the catalyst B1 was molded so as to achieve a loading ratio of 60% by mass by using a spherical material with a diameter of 4.0 mm mainly composed of alumina and silica as an inert carrier. A reaction rate k of the catalyst B2 was 0.43.

(Catalyst C1)

A pre-calcined powder with an atomic ratio of Mo:Bi:Fe:Co:Ni:K = 12:1.8:1.9:5.0:2.6:0.090 was obtained by changing the formulation of the raw material in the production of the catalyst A1. A catalyst C1 with a diameter of 4.80 mm was obtained in exactly the same manner as the catalyst A1, except that this pre-calcined powder was molded so as to achieve a loading ratio of 40% by mass by using a spherical material with a diameter of 4.4 mm mainly composed of alumina and silica as an inert carrier. A reaction rate k of the catalyst C1 was 0.42.

(Catalyst D1)

A pre-calcined powder with an atomic ratio of Mo:Bi:Fe:Co:Ni:K = 12:0.6:2.1:5.7:3.0:0.090 was obtained by, in the production of the catalyst A1, adding 60 wt% of nitric acid before adding the preparation solution 2 during the preparation process in order to adjust the pH of the preparation solution to 4.0, and by changing the formulation of the raw material. This pre-calcined powder was processed in exactly the same manner in the production of the catalyst A1 to obtain a catalyst D1 with a diameter of 5.30 mm. A reaction rate k of the catalyst D1 was 0.62.

(Catalyst E1)

A pre-calcined powder with an atomic ratio of Mo:Bi:Fe:Co:Ni:K = 12:0.8:2.1:6.6:2.2:0.040 was obtained by changing the formulation of the raw material in the production of the catalyst A1. A catalyst E1 with a diameter of 5.40 mm was obtained in exactly the same manner as in the production of the catalyst A1. A reaction rate k of the catalyst E1 was 0.60.

[Example 1]

In a stainless steel reactor with an inner diameter of 25 mm provided with a jacket for circulating molten salt as a heat transfer medium and a thermocouple for measuring catalyst layer temperature on a tube axis, a thermowell with an outer diameter of 3 mm was installed for the thermocouple, and the followings were packed from an inlet side for a raw material gas to an outlet of the gas: as an upper layer (on the inlet side for the raw material gas), 120 cm of a diluted catalyst obtained by mixing a catalyst B1 and a silica-alumina-mixture inert spherical carrier in a mass ratio of 70:30 (70% by mass dilution, the same calculation will be repeated hereafter); as a middle layer, 120 cm of the non-diluted catalyst B1; and as a lower layer, 160 cm of a non-diluted catalyst A1. akt/akn was 1.71. In this way, three catalyst layers were provided. A reaction bath temperature was set to 315°C, and supply amounts of propylene, air, water, and nitrogen were set to obtain a raw material molar ratio of propylene:oxygen (oxygen contained in supplied air):water:nitrogen (nitrogen supplied separately from air) = 1:1.4:1.4:1.6. These feed materials were flowed so that space velocity of propylene was 190 $hr^{-1}$, and the reaction was started with a pressure at the outlet side of a reaction tube being 110 kPaG when all the gas was flowing. After 300 hours, the reaction bath temperature was changed and the oxidation reaction of propylene was carried out. The results of investigating the reaction results by changing the reaction bath temperature are shown in Table 1. A peak temperature of each catalyst layer refers to a temperature at the hottest point in the temperature profile in the reaction tube obtained by measuring the temperature at predetermined intervals (in this Example, intervals of 5 cm). Note that, rows in which there is no peak temperature or reaction result for each layer in A1 and A2 in Table 1 means that A1 and A2 are calculated values calculated by interpolation or extrapolation of the measured data (the same applies below). In addition, a calculation process for Sf/St at BT of 330°C, at which a yield of acrolein is the highest, is shown in Table 1-2.

[Table 1]

| BT [°C] | | First Layer Peak Temperature [°C] | Second Layer Peak Temperature [°C] | Third Layer Peak Temperature [°C] | Propylene Conversion Rate [%] | Acrolein Yield [%] | Sf/St | ΔPTf/ΔBT | A2-A1 [°C] |
|---|---|---|---|---|---|---|---|---|---|
| | 320 | 375 | 377 | 346 | 78.8 | 71.1 | 20.1 | | |
| | 324 | 382 | 381 | 353 | 87.2 | 78.1 | 25.1 | | |
| A1 | 327 | | | | | 81.3 | | 1.50 | 14 |
| | 327 | 388 | 384 | 367 | 94.5 | 82.0 | 30.3 | | |
| A | 330 | 393 | 387 | 376 | 95.9 | 82.3 | 30.4 | | |
| | 334 | 399 | 391 | 381 | 96.7 | 81.9 | 29.8 | | |
| | 338 | 407 | 395 | 384 | 97.3 | 82.1 | 28.7 | | |
| A2 | 341 | | | | | 81.3 | | | |

[Table 1-2]

| Catalyst Layer | Sum of Spot Heat Generation Tempe-ratures in First Layer [°C] | First Layer | | | | | | | | | | | | | | | | | | | | | | | | |
|---|---|---|---|---|---|---|---|---|---|---|---|---|---|---|---|---|---|---|---|---|---|---|---|---|---|---|
| Temperature Distribution Measurement Position [cm] | | 0 | 5 | 10 | 15 | 20 | 25 | 30 | 35 | 40 | 45 | 50 | 55 | 60 | 65 | 70 | 75 | 80 | 85 | 90 | 95 | 100 | 105 | 110 | 115 | 120 |
| Spot Temperature T1 [°C] | | 245 | 250 | 280 | 300 | 318 | 331 | 343 | 354 | 366 | 373 | 381 | 388 | 392 | 392 | 393 | 393 | 390 | 389 | 385 | 382 | 379 | 377 | 376 | 376 | 376 |
| Reaction Bath Temperature [°C] | | 330 | 330 | 330 | 330 | 330 | 330 | 330 | 330 | 330 | 330 | 330 | 330 | 330 | 330 | 330 | 330 | 330 | 330 | 330 | 330 | 330 | 330 | 330 | 330 | 330 |
| Spot Heat Generation Temperature T2 [°C] | 935 | 0 | 0 | 0 | 0 | 0 | 1 | 13 | 24 | 36 | 43 | 51 | 58 | 62 | 62 | 63 | 63 | 60 | 59 | 55 | 52 | 49 | 47 | 46 | 46 | 46 |

| Catalyst Layer | Sum of Spot Heat Generation Temperatures in Second Layer [°C] | Second Layer | | | | | | | | | | | | | | | | | | | | | | | |
|---|---|---|---|---|---|---|---|---|---|---|---|---|---|---|---|---|---|---|---|---|---|---|---|---|---|
| Temperature Distribution Measurement Position [cm] | | 125 | 130 | 135 | 140 | 145 | 150 | 155 | 160 | 165 | 170 | 175 | 180 | 185 | 190 | 195 | 200 | 205 | 210 | 215 | 220 | 225 | 230 | 235 | 240 |
| Spot Temperature T1 [°C] | | 379 | 383 | 383 | 386 | 387 | 386 | 384 | 381 | 379 | 379 | 374 | 372 | 370 | 368 | 365 | 364 | 362 | 360 | 359 | 358 | 357 | 356 | 355 | 356 |
| Reaction Bath Temperature [°C] | | 330 | 330 | 330 | 330 | 330 | 330 | 330 | 330 | 330 | 330 | 330 | 330 | 330 | 330 | 330 | 330 | 330 | 330 | 330 | 330 | 330 | 330 | 330 | 330 |
| Spot Heat Generation Temperature T2 [°C] | 983 | 49 | 53 | 53 | 56 | 57 | 56 | 54 | 51 | 49 | 49 | 44 | 42 | 40 | 38 | 35 | 34 | 32 | 30 | 29 | 28 | 27 | 26 | 25 | 26 |

| Catalyst Layer | Sum of Spot Heat Generation Temperatures in Third Layer [°C] | Third Layer | | | | | | | | | | | | | | | |
|---|---|---|---|---|---|---|---|---|---|---|---|---|---|---|---|---|---|
| Temperature Distribution Measurement Position [cm] | | 245 | 250 | 255 | 260 | 265 | 270 | 275 | 280 | 285 | 290 | 295 | 300 | 305 | 310 | 315 | 320 |
| Spot Temperature T1 [°C] | | 360 | 368 | 372 | 376 | 375 | 375 | 372 | 373 | 372 | 368 | 367 | 363 | 360 | 357 | 355 | 354 |
| Reaction Bath Temperature [°C] | | 330 | 330 | 330 | 330 | 330 | 330 | 330 | 330 | 330 | 330 | 330 | 330 | 330 | 330 | 330 | 330 |
| Spot Heat Generation Temperature T2 [°C] | 837 | 30 | 38 | 42 | 46 | 45 | 45 | 42 | 43 | 42 | 38 | 37 | 33 | 30 | 27 | 25 | 24 |

| Catalyst Layer | Sum of Spot Heat Generation Temperatures in Third Layer [°C] | Third Layer | | | | | | | | | | | | | | | |
|---|---|---|---|---|---|---|---|---|---|---|---|---|---|---|---|---|---|
| Temperature Distribution Measurement Position [cm] | | 325 | 330 | 335 | 340 | 345 | 350 | 355 | 360 | 365 | 370 | 375 | 380 | 385 | 390 | 395 | 400 |
| Spot Temperature T1 [°C] | | 352 | 351 | 350 | 349 | 348 | 348 | 347 | 347 | 346 | 345 | 343 | 342 | 342 | 341 | 341 | 341 |
| Reaction Bath Temperature [°C] | | 330 | 330 | 330 | 330 | 330 | 330 | 330 | 330 | 330 | 330 | 330 | 330 | 330 | 330 | 330 | 330 |
| Spot Heat Generation Temperature T2 [°C] | 837 | 22 | 21 | 20 | 19 | 18 | 18 | 17 | 17 | 16 | 15 | 13 | 12 | 12 | 11 | 11 | 11 |

As a result, the sum of the spot heat generation temperatures in the first layer = 935, the sum of the spot heat generation temperatures in the second layer = 983, and the sum of the spot heat generation temperatures in the third layer = 837, so that sf/st is 837 / (935 + 983 + 837) × 100 = 30.4.

[Example 2]

The reaction was started in exactly the same manner as in Example 1 except that the followings were packed: as an upper layer (on the inlet side for the raw material gas), 120 cm of a diluted catalyst obtained by mixing the catalyst B1 and a silica-alumina-mixture inert spherical carrier in a mass ratio of 80:20; as a middle layer, 120 cm of the non-diluted catalyst B1; and as a lower layer, 160 cm of the non-diluted catalyst A1 (akt/akn = 1.75), and the reaction results were investigated. The results are shown in Table 2.

[Table 2]

| BT [°C] | | First Layer Peak Temperature [°C] | Second Layer Peak Temperature [°C] | Third Layer Peak Temperature [°C] | Propylene Conversion Rate [%] | Acrolein Yield [%] | Sf/St | ΔPTf/ΔBT | A2-A1 [°C] |
|---|---|---|---|---|---|---|---|---|---|
| A1 | 318 | | | | | 80.8 | | | |
| | 332 | 406 | 381 | 372 | 96.5 | 81.6 | 28.5 | | |
| A | 334 | 406 | 383 | 376 | 96.8 | 81.8 | 28.9 | 1.81 | 20 |
| | 335 | 407 | 384 | 377 | 96.9 | 81.4 | 29.1 | | |
| A2 | 338 | 406 | 387 | 383 | 97.0 | 80.8 | 30.6 | | |

[Example 3]

The followings were packed: as an upper layer (on the inlet side for the raw material gas), 120 cm of a diluted catalyst obtained by mixing the catalyst B1 and a silica-alumina-mixture inert spherical carrier in a mass ratio of 70:30; as a middle layer, 180 cm of the non-diluted catalyst B1; and as a lower layer, 100 cm of the non-diluted catalyst A1 (akt/akn = 2.47). In addition, as reaction conditions, supply amounts of propylene, air, water, and nitrogen were set to obtain a raw material molar ratio of propylene:oxygen (oxygen contained in supplied air):water:nitrogen (nitrogen supplied separately from air) = 1:1.4:1.4:1.6. These feed materials were flowed so that space velocity of propylene was 130 hr$^{-1}$), and a pressure at the outlet side of a reaction tube was 85 kPaG when all the gas was flowing. The reaction was started in exactly the same manner as in Example 1 except for the above conditions, and the reaction results were investigated. The results are shown in Table 3.

[Table 3]

| BT [°C] | | First Layer Peak Temperature [°C] | Second Layer Peak Temperature [°C] | Third Layer Peak Temperature [°C] | Propylene Conversion Rate [%] | Acrolein Yield [%] | Sf/St | ΔPTf/ΔBT | A2-A1 [°C] |
|---|---|---|---|---|---|---|---|---|---|
| | 321 | 380 | 370 | 334 | 89.5 | 78.9 | 10.4 | | |
| A1 | 324 | | | | | 81.2 | | | |
| A | 325 | 389 | 373 | 344 | 95.0 | 82.2 | 13.5 | | |
| | 329 | 398 | 377 | 349 | 96.4 | 82.1 | 13.4 | 1.25 | 11 |
| | 333 | 406 | 380 | 353 | 97.2 | 81.4 | 13.2 | | |
| A2 | 335 | | | | | 81.2 | | | |

[Example 4]

A thermowell with an outer diameter of 6 mm for a thermocouple was inserted into a stainless steel reactor with an inner diameter of 27 mm, and silica-alumina spheres with a diameter of 5.2 mm were packed from the inlet side for the raw material gas until the packing length of 15 cm was achieved. The followings were packed: as an upper layer (on the inlet side for the raw material gas), 77 cm of a diluted catalyst obtained by mixing the catalyst B1 and a silica-alumina-mixture inert spherical carrier in a mass ratio of 85:15; as a middle layer, 77 cm of the non-diluted catalyst B1; and as a lower layer, 176 cm of the non-diluted catalyst A1 (akt/akn = 1.45). In addition, as reaction conditions, supply amounts of propylene, air, water, and nitrogen were set to obtain a raw material molar ratio of propylene: oxygen (oxygen contained in supplied air):water:nitrogen (nitrogen supplied separately from air) = 1:1.7:1:2.4. These feed materials were flowed so that space velocity of propylene was 100 hr$^{-1}$), a pressure at the outlet side of a reaction tube was 35 kPaG when all the gas was flowing. The reaction was started in exactly the same manner as in Example 1 except for the above conditions, and the reaction results were investigated. The results are shown in Table 4.

[Table 4]

| BT [°C] | | First Layer Peak Temperature [°C] | Second Layer Peak Temperature [°C] | Third Layer Peak Temperature [°C] | Propylene Conversion Rate [%] | Acrolein Yield [%] | Sf/St | ΔPTf/ΔBT | A2-A1 [°C] |
|---|---|---|---|---|---|---|---|---|---|
| | 315 | 374 | 366 | 337 | 84.2 | 77.6 | 28.9 | | |
| A1 | 317 | | | | | 85.2 | | | |
| | 318 | 380 | 370 | 341 | 93.7 | 85.3 | 33.6 | | |
| A | 324 | 392 | 377 | 360 | 98.0 | 86.2 | 34.2 | 2.64 | 13 |
| | 327 | 398 | 381 | 364 | 98.4 | 85.7 | 33.4 | | |
| A2 | 330 | | | | | 85.2 | | | |
| | 330 | 403 | 384 | 367 | 98.7 | 85.1 | 32.5 | | |

[Example 5]

A thermowell with an outer diameter of 3 mm for a thermocouple was inserted into a stainless steel reactor with an inner diameter of 21 mm, and silica-alumina spheres with a diameter of 5.2 mm were packed from the inlet side for the raw material gas until the packing length of 20 cm was achieved. The followings were packed: as an upper layer (on the inlet side for the raw material gas), 200 cm of a non-diluted catalyst C1; and as a lower layer, 150 cm of a non-diluted catalyst D1 (aktlakn = 1.89). In addition, as reaction conditions, supply amounts of propylene, air, water, and nitrogen were set to obtain a raw material molar ratio of propylene:oxygen (oxygen contained in supplied air):water:nitrogen (nitrogen supplied separately from air) = 1: 1.75:0.6:4.35. These feed materials were flowed so that space velocity of propylene was 180 hr$^{-1}$), and a pressure at the outlet side of a reaction tube was 90 kPaG when all the gas was flowing. The reaction was started in exactly the same manner as in Example 1 except for the above conditions, and the reaction results were investigated. The results are shown in Table 5.

[Table 5]

| BT [°C] | | First Layer Peak Temperature [°C] | Second Layer Peak Temperature [°C] | Third Layer Peak Temperature [°C] | Propylene Conversion Rate [%] | Acrolein Yield [%] | Sf/ST | ΔPTf/ΔBT | A2-A1 [°C] |
|---|---|---|---|---|---|---|---|---|---|
| | 334 | 392 | 352 | - | 93.8 | 83.4 | 24.6 | | |
| A1 | 335 | | | - | | 83.7 | | | |
| | 336 | 407 | 350 | - | 95.6 | 84.5 | 19.7 | | |
| A | 338 | 415 | 351 | - | 96.2 | 84.7 | 18.2 | 0.75 | 14 |
| | 341 | 425 | 353 | - | 96.9 | 84.6 | 17.0 | | |
| | 344 | 434 | 356 | - | 97.4 | 84.2 | 15.8 | | |
| A2 | 349 | | | - | | 83.7 | | | |

[Comparative Example 1]

The reaction was started in exactly the same manner as in Example 1 except that the followings were packed: as an upper layer (on the inlet side for the raw material gas), 75 cm of a diluted catalyst obtained by mixing the catalyst B1 and a silica-alumina-mixture inert spherical carrier in a mass ratio of 75:25; as a middle layer, 75 cm of the non-diluted catalyst B1; and as a lower layer, 250 cm of the non-diluted catalyst A1 (akt/akn = 1.29), and the reaction results were investigated. The results are shown in Table 6.

[Table 6]

| | BT [°C] | First Layer Peak Temperature [°C] | Second Layer Peak Temperature [°C] | Third Layer Peak Temperature [°C] | Propylene Conversion Rate [%] | Acrolein Yield [%] | Sf/St | PTf/BT | A2-A1 [°C] |
|---|---|---|---|---|---|---|---|---|---|
| | 318 | 399 | 406 | 363 | 80.4 | 71.5 | 44.5 | | |
| | 319 | 401 | 407 | 365 | 82.1 | 72.8 | 43.9 | | |
| | 321 | 406 | 411 | 374 | 92.7 | 80.9 | 34.8 | | |
| A1 | 321 | | | | | 81.0 | | | |
| A | 322 | 407 | 412 | 377 | 96.0 | 82.0 | 42.4 | 3.00 | 4 |
| | 324 | 414 | 418 | 383 | 97.0 | 81.4 | 41.1 | | |
| A2 | 325 | | | | | 81.0 | | | |

[Comparative Example 2]

The reaction was started in exactly the same manner as in Example 1 except that the followings were packed: as an upper layer (on the inlet side for the raw material gas), 75 cm of a diluted catalyst obtained by mixing a catalyst B2 and a silica-alumina-mixture inert spherical carrier in a mass ratio of 85:15; as a middle layer, 75 cm of the non-diluted catalyst B2; and as a lower layer, 250 cm of a non-diluted catalyst E1 (akt/akn = 1.40), and the reaction results were investigated. The results are shown in Table 7.

[Table 7]

| | BT [°C] | First Layer Peak Temperature [°C] | Second Layer Peak Temperature [°C] | Third Layer Peak Temperature [°C] | Propylene Conversion Rate [%] | Acrolein Yield [%] | Sf/St | ΔPTf/ΔBT | A2-A1 [°C] |
|---|---|---|---|---|---|---|---|---|---|
| | 318 | 387 | 395 | 354 | 78.2 | 69.2 | 33.6 | | |
| | 321 | 392 | 400 | 356 | 82.9 | 72.9 | 35.6 | | |
| A1 | 324 | | | | | 80.0 | | | |
| | 324 | 402 | 406 | 362 | 93.3 | 80.2 | 41.4 | | |
| A | 325 | 402 | 409 | 373 | 95.7 | 81.0 | 42.4 | 5.64 | 9 |
| | 327 | 406 | 412 | 380 | 96.7 | 80.8 | 43.4 | | |
| A2 | 333 | | | | | 80.0 | | | |

[Comparative Example 3]

The reaction was started in exactly the same manner as in Example 1 except that the followings were packed: as an upper layer (on the inlet side for the raw material gas), 75 cm of a diluted catalyst obtained by mixing the catalyst B1 and a silica-alumina-mixture inert spherical carrier in a mass ratio of 85:15; as a middle layer, 75 cm of the non-diluted catalyst B1; and as a lower layer, 250 cm of the non-diluted catalyst A1 (akt/akn = 1.31), and the reaction results were investigated. The results are shown in Table 8.

# EP 4 603 472 A1

[Table 8]

| | BT [°C] | First Layer Peak Temperature [°C] | Second Layer Peak Temperature [°C] | Third Layer Peak Temperature [°C] | Propylene Conversion Rate [%] | Acrolein Yield [%] | Sf/St | ΔPTf/ΔBT | A2-A1 [°C] |
|---|---|---|---|---|---|---|---|---|---|
| | 314 | 386 | 391 | 350 | 88.5 | 77.6 | 38.3 | | |
| | 315 | 393 | 395 | 350 | 89.8 | 78.5 | 37.2 | | |
| A1 | 316 | | | | | 81.3 | | | |
| A | 316 | 393 | 399 | 360 | 95.9 | 82.3 | 43.0 | 22.00 | 1 |
| A2 | 317 | 394 | 399 | 382 | 98.1 | 81.3 | 45.6 | | |

The results described above are summarized in Table 9. It can be seen from Table 9 that ΔPTf/ΔBT is significantly reduced by widening the operation window (A2 - A1). This is particularly beneficial in the method for producing acrolein from propylene, because it leads to stable operation of a plant under the above-mentioned restrictions on the reactor and process. Similarly, it can also be seen that ΔPTf/ΔBT is significantly reduced by setting Sf/St below a specific value at the reaction bath temperature at which the yield of acrolein is the highest. Thus, it can be seen that the operation window and Sf/St can be controlled by akt/akn as described above.

[Table 9]

| | akt/akn | A1[°C] | A2[°C] | A2-A1[°C] | Sf/St | ΔPTf/ΔBT | Maximum Acrolein Yield [%] |
|---|---|---|---|---|---|---|---|
| Ex. 1 | 1.71 | 327 | 341 | 14 | 30.4 | 1.50 | 82.3 |
| Ex. 2 | 1.75 | 318 | 338 | 20 | 28.9 | 1.81 | 81.8 |
| Ex. 3 | 2.47 | 324 | 335 | 11 | 13.5 | 1.25 | 82.2 |
| Ex. 4 | 1.45 | 317 | 330 | 13 | 34.2 | 2.64 | 86.2 |
| Ex. 5 | 1.89 | 335 | 349 | 14 | 18.2 | 0.75 | 84.7 |
| Comp. Ex. 1 | 1.29 | 321 | 325 | 4 | 42.4 | 3.00 | 82.0 |
| Comp. Ex. 2 | 1.40 | 324 | 333 | 9 | 43.2 | 5.64 | 81.0 |
| Comp. Ex. 3 | 1.31 | 316 | 317 | 1 | 43.0 | 22.00 | 82.3 |

The present application is based on Japanese Patent Application No. 2022-163792 filed on October 12, 2022, the content of which is incorporated herein by reference.

INDUSTRIAL APPLICABILITY

The present invention can improve the yield and suppress runaway reactions in unsaturated aldehyde production plants. This makes it possible to maintain a stable yield and to achieve stable operation in the industrial plants for a long period of time.

REFERENCE NUMERALS

10      Multitubular Reactor
20      Reactor Tube
21,22,23   Catalyst Layer

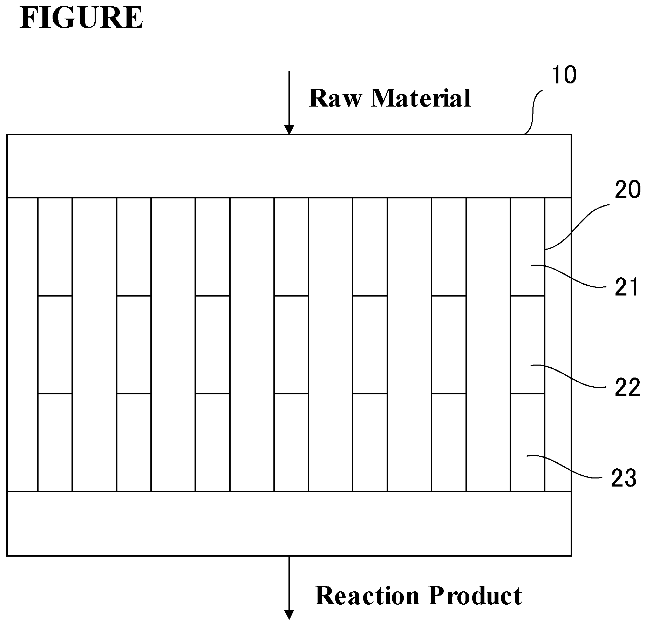

## Claims

1. A method for producing an unsaturated aldehyde corresponding to an alkene comprising partially oxidizing the alkene using a fixed-bed multitubular reactor, wherein

the fixed-bed multitubular reactor comprises multiple reaction tubes and a reaction bath for adjusting temperature of the multiple reaction tubes,

the reaction tube is provided with two or more catalyst layers in a gas flow direction, and

when a reaction bath temperature at which a yield of the unsaturated aldehyde is the highest is defined as A (°C), and reaction bath temperatures at which the yield is 1.0% lower than the highest value are defined as A1 (°C) and A2 (°C), following formulae (1) and (2) hold:

$$A1 < A < A2 \quad (1)$$

$$(A2 - A1) \geq 10 \quad (2).$$

2. The method for producing the unsaturated aldehyde according to claim 1, wherein

regarding Sf and St when the unsaturated aldehyde is produced at the reaction bath temperature A (°C), a following formula (3) holds:

$$(Sf/St) \times 100 \leq 42.0 \quad (3),$$

and Sf and St are determined by following steps (a) to (c):

(a) p (p is an integer of 2 or more) thermocouples are arranged at regular intervals throughout the gas flow direction of the two or more catalyst layers provided in the reaction tube, and spot temperatures $T1_j$ (°C) (j is 1 to p) are obtained by using the thermocouples at p measurement points in the reaction tube when producing the unsaturated aldehyde at the reaction bath temperature A (°C);

(b) for each of the measurement points, a spot heat generation temperature $T2_j$, which is a value ($T1_j$ - A) obtained by subtracting the reaction bath temperature A (°C) from the spot temperature $T1_j$ (°C), is calculated, provided that if $T1_j$ - A is a negative value, the spot heat generation temperature $T2_j$ of the relevant measurement point is set to 0; and

(c) St is defined as a value of the sum of the spot heat generation temperatures $T2_j$ at all the measurement points in the reaction tube, and Sf is defined as a value of the sum of the spot heat generation temperatures $T2_k$ at the measurement points provided in the catalyst layer closest to an outlet, where k is 1 to q, and q is number of the measurement points provided in the catalyst layer closest to the outlet, q < p.

3. The method for producing the unsaturated aldehyde according to claim 1 or 2, wherein

a catalytically active component contained in the catalyst layer closest to an inlet of a reaction raw material gas has composition represented by a following formula (I-1):

$$Mo_{a1}Bi_{b1}Ni_{c1}CO_{d1}Fe_{e1}X_{f1}Cs_{g1}Z_{h1}O_{i1} \quad (I\text{-}1),$$

where, Mo, Bi, Ni, Co, Fe, Cs, and O represent molybdenum, bismuth, nickel, cobalt, iron, cesium, and oxygen, respectively;

X represents at least one element selected from tungsten, antimony, tin, zinc, chromium, manganese, magnesium, silicon, aluminum, cerium, and titanium;

Z represents at least one element belonging to groups 1 to 16 on the periodic table and selected from elements other than Mo, Bi, Ni, Co, Fe, Cs, O, and X described above; and

a1, b1, c1, d1, e1, f1, g1, h1, and i1 represent number of atoms of molybdenum, bismuth, nickel, cobalt, iron, X, Cs, Z, and oxygen, respectively, wherein, when a1 = 12, $0 < b1 \leq 7.0$, $0 \leq c1 \leq 10$, $0 < d1 \leq 10$, $0 < e1 \leq 5.0$, $0 \leq f1 \leq 2.0$, $0 < g1 \leq 3.0$, and $0 \leq h1 \leq 5.0$ are satisfied, and i1 is a value determined by an oxidation state of each element.

4. The method for producing the unsaturated aldehyde according to claim 1 or 2, wherein

a catalytically active component contained in the catalyst layer closest to the outlet of the reaction raw material gas has composition represented by a following formula (I-2):

$$Mo_{a2}Bi_{b2}Ni_{c2}Co_{d2}Fe_{e2}X_{f2}K_{g2}Z_{h2}O_{i2} \quad (I\text{-}2)$$

where, Mo, Bi, Ni, Co, Fe, and K represent molybdenum, bismuth, nickel, cobalt, iron, and potassium, respectively;

X represents at least one element selected from tungsten, antimony, tin, zinc, chromium, manganese, magnesium, silicon, aluminum, cerium, and titanium;

Z represents at least one element belonging to groups 1 to 16 on the periodic table and selected from elements other than Mo, Bi, Ni, Co, Fe, K, O, and X described above; and

a2, b2, c2, d2, e2, f2, g2, h2, and i2 represent number of atoms of molybdenum, bismuth, nickel, cobalt, iron, X, K, Z, and oxygen, respectively, wherein, when $a2 = 12$, $0 < b2 \leq 7.0$, $0 \leq c2 \leq 10$, $0 < d2 \leq 10$, $0 < e2 \leq 5.0$, $0 \leq f2 \leq 2.0$, $0 \leq g2 \leq 3.0$, and $0 \leq h2 \leq 5.0$ are satisfied, and i2 is a value determined by an oxidation state of each element.

5. The method for producing the unsaturated aldehyde according to claim 1 or 2, wherein
a reaction bath temperature is 310°C or higher and 340°C or lower.

6. The method for producing the unsaturated aldehyde according to claim 1 or 2, wherein
a volume ratio of oxygen to the alkene (oxygen/alkene) in a reaction raw material gas is 1.0 or more and 1.8 or less.

7. The method for producing the unsaturated aldehyde according to claim 1 or 2, wherein
the reaction tube does not have an inert layer closer to the inlet than the catalyst layers.

8. The method for producing the unsaturated aldehyde according to claim 1 or 2, wherein

the reaction tube is provided with three catalyst layers in the gas flow direction, and
a ratio of the sum of packing lengths of a first layer and a second layer counted from an inlet side of the reaction tube to a packing length of a third layer ((packing length of first layer + packing length of second layer)/packing length of third layer) is 1.5 or more and 3.5 or less.

9. The method for producing the unsaturated aldehyde according to claim 1 or 2, wherein

the reaction tube is provided with two or more catalyst layers in the gas flow direction, and a type, dilution ratio, and packing length of the catalyst in each of the catalyst layers are set so that akt/akn defined by following formulae (II), (III), and (IV) is 1.41 or more and 10.00 or less:

$$\text{Reaction rate k of catalyst} = -\text{Ln}\,(1 - x/100) \qquad (II)$$

Actual reaction rate ak of catalyst layer = (dilution ratio of relevant catalyst layer) $\times$ (packing length of relevant catalyst layer) $\times$ (reaction rate k of catalyst in relevant catalyst layer) $\qquad$ (III)

akt/akn = (value of the sum of actual reaction rates ak of all catalyst layers)/(actual reaction rate ak of catalyst layer closest to outlet of reaction tube) $\qquad$ (IV)

wherein x represents a raw material gas conversion ratio (%) when the catalyst is packed into a differential reactor and a partial oxidation reaction of the alkene is conducted at the reaction bath temperature of 360°C.

10. A production device of an unsaturated aldehyde by partially oxidizing an alkene to produce a corresponding unsaturated aldehyde, comprising:

a fixed-bed multitubular reactor including multiple reaction tubes; and
a reaction bath for adjusting temperature of the multiple reaction tubes, wherein
the reaction tube is provided with two or more catalyst layers in a gas flow direction, and
when a reaction bath temperature at which a yield of the unsaturated aldehyde is the highest is defined as A (°C), and reaction bath temperatures at which the yield is 1.0% lower than the highest value are defined as A1 (°C) and A2 (°C), following formulae (1) and (2) hold:

$$A1 < A < A2 \qquad (1)$$

$$(A2 - A1) \geq 10 \quad (2).$$

**FIGURE**

## INTERNATIONAL SEARCH REPORT

| International application No. |
| --- |
| **PCT/JP2023/036202** |

### A. CLASSIFICATION OF SUBJECT MATTER

*C07C 45/35*(2006.01)i; *B01J 23/887*(2006.01)i; *B01J 35/51*(2024.01)i; *C07B 61/00*(2006.01)i; *C07C 47/22*(2006.01)i
FI: C07C45/35; C07C47/22 G; B01J35/08 Z; B01J23/887 Z; C07B61/00 300

According to International Patent Classification (IPC) or to both national classification and IPC

### B. FIELDS SEARCHED

Minimum documentation searched (classification system followed by classification symbols)

C07C45/35; B01J23/887; B01J35/08; C07B61/00; C07C47/22

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | WO 2019/198763 A1 (NIPPON KAYAKU KABUSHIKI KAISHA) 17 October 2019 (2019-10-17) claims, examples | 1-7, 9-10 |
| Y | claims, examples | 8 |
| Y | WO 2020/203266 A1 (NIPPON KAYAKU KABUSHIKI KAISHA) 08 October 2020 (2020-10-08) claims, examples | 8 |
| A | claims, examples | 1-7, 9-10 |
| A | JP 2010-077087 A (TOAGOSEI CO., LTD.) 08 April 2010 (2010-04-08) claims, examples | 1-10 |

☐ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **06 December 2023** | **19 December 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/036202**

| Patent document cited in search report | | | Publication date (day/month/year) | Patent family member(s) | | | Publication date (day/month/year) |
|---|---|---|---|---|---|---|---|
| WO | 2019/198763 | A1 | 17 October 2019 | US<br>claims, examples<br>EP<br>CN | 2021/0155569<br><br>3778541<br>111936458 | A1<br><br>A1<br>A | |
| WO | 2020/203266 | A1 | 08 October 2020 | US<br>claims, examples<br>EP<br>CN | 2022/0169587<br><br>3950128<br>113573811 | A1<br><br>A1<br>A | |
| JP | 2010-077087 | A | 08 April 2010 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003164763 A
- JP 2003146920 A
- WO 2014181839 A
- WO 2016136882 A
- JP H10168003 A
- JP 2004002209 A
- JP 2001328951 A
- JP 2005320315 A
- JP H083093 A
- JP 2001226302 A
- JP 6912153 B
- JP 2022163792 A